(19) Europäisches Patentamt
European Patent Office
Office européen des brevets

(11) **EP 2 605 810 B1**

(12) **EUROPÄISCHE PATENTSCHRIFT**

(45) Veröffentlichungstag und Bekanntmachung des Hinweises auf die Patenterteilung:
**08.10.2014 Patentblatt 2014/41**

(21) Anmeldenummer: **11769766.4**

(22) Anmeldetag: **17.08.2011**

(51) Int Cl.:
***A61M 1/16*** *(2006.01)*

(86) Internationale Anmeldenummer:
**PCT/DE2011/001606**

(87) Internationale Veröffentlichungsnummer:
**WO 2012/022304 (23.02.2012 Gazette 2012/08)**

(54) **VORRICHTUNG ZUR EXTRAKORPORALEN BLUTBEHANDLUNG**

APPARATUS FOR EXTRACORPOREAL BLOOD TREATMENT

DISPOSITIF DE TRAITEMENT EXTRACORPOREL DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.08.2010 DE 102010034626**

(43) Veröffentlichungstag der Anmeldung:
**26.06.2013 Patentblatt 2013/26**

(73) Patentinhaber: **B. Braun Avitum AG**
**34212 Melsungen (DE)**

(72) Erfinder:
• **MEIBAUM, Jörn**
**34225 Baunatal (DE)**
• **MOLL, Stefan**
**34212 Melsungen (DE)**
• **CASTELLARNAU, Alex**
**34212 Melsungen (DE)**

(74) Vertreter: **Arth, Hans-Lothar**
**ABK Patent Attorneys**
**Jasminweg 9**
**14052 Berlin (DE)**

(56) Entgegenhaltungen:
**EP-A1- 2 005 982     WO-A1-99/62574**
**DE-A1- 2 934 190     US-A- 5 772 606**
**US-A1- 2004 204 634**

**Beschreibung**

Die Erfindung bezieht sich auf eine Vorrichtung zur extrakorporalen Blutbehandlung zur Unterscheidung urämischer Toxine im Abfluss der Vorrichtung.

Bei Patienten mit eingeschränkter oder fehlender Nierenfunktion werden Abfallprodukte des natürlichen Stoffwechsels einschließlich urämischer Toxine durch Nierenersatz- bzw. Dialyseverfahren entfernt. Dabei erfolgt die Entfernung der Stoffe aus dem Blut, welches dem Patienten entnommen und extrakorporal geführt wird, durch den Kontakt des Blutes mit einer Dialysierflüssigkeit, wobei Blut und Dialysierflüssigkeit nicht direkt, sondern über eine Membran miteinander in Kontakt stehen. Die Dialysierflüssigkeit ist mit verschiedenen Salzen versetzt und ruft somit diffusive und konvektive Effekte hervor, die für den Stofftransport vom Blut in die Dialysierflüssigkeit über die extrakorporal angeordnete Membran verantwortlich sind. Nach erfolgter Entfernung eines Teils der Abfallstoffe wird das so behandelte Blut dem Patienten wieder zu geführt.

Zum Test eines Dialyseapparats, Teilen eines Dialyseapparats oder geänderter Dialyseparameter werden die Konzentrationen urämischer Toxine vor und nach einer Dialysetherapie bestimmt. Die Reduktion der jeweiligen Stoffe stellt dabei die zentrale Basis zur Bewertung der Dialysedosis dar.

Ein gängiges Markerelement ist Harnstoff, welcher auch Urea genannt wird. Entsprechend gilt die Harnstoffreduktionsrate als entscheidender Parameter in der Dialysetechnik. Die Bestimmung der Harnstoffreduktion kann auf unterschiedlichen Wegen erfolgen.

Eine klassische Methode stellt die chemische Bestimmung der Harnstoffkonzentration im Blut jeweils vor und nach einer Dialysetherapie dar. Das Problem dieser Methode ist jedoch, dass die Blutproben dem Patienten entnommen und zu einem Labor geschickt werden müssen, welches für die Bestimmung der Harnstoffkonzentration entsprechend ausgerüstet ist. Dieser Vorgang kann durchaus mehrere Tage in Anspruch nehmen.

Des Weiteren kann die Harnstoffkonzentration bzw. deren Änderung durch die Bestimmung der Leitfähigkeit im Dialysat ermittelt werden. Ein Produkt am Markt, welches nach diesem Prinzip arbeitet, ist das Produkt Biostat© Urea Monitor der Firma Baxter. Die Problematik bei der Messung mit Leitfähigkeit ist, dass Leitfähigkeitsänderung auch durch andere Einflussfaktoren erfolgen kann, wie z.B. durch pH-Änderungen und die Messung somit unter Umständen verfälscht wird.

Eine dritte Möglichkeit zur Bestimmung der Dialysedosis ist die Messung der Harnsäurereduktion, welche - wie allgemein bekannt - im Wesentlichen der Harnstoffreduktion entspricht, über eine Dialysetherapie mittels UV-Absorptionsmessungen im Abfluss des Dialysates. Uhlin hat in seiner Dissertation mit dem Thema "Haemodialysis Treatment monitored on-line by ultra violet absorbance" [Linköping University Medical Dissertation No 962, 2006] gezeigt, dass die Absorbanzänderung im abfließenden Dialysat bei 280 nm eine sehr gute Korrelation zur Konzentrationsänderung von Harnstoff im Blut des Patienten darstellt. Eine solche Messeinrichtung wird in der EP 1 083 948 B1 beschrieben. In diesem Stand der Technik ist sowohl der Aufbau als auch die Position des Sensors für dialysetechnische Anwendungen beschrieben.

Allerdings ist es mit den aus dem Stand der Technik bekannten Vorrichtungen und Verfahren nicht möglich, zeitnah zur bzw. während der Therapie des Patienten eine Differenzierung anderer Abfallstoffe bzw. toxischer Substanzen von Harnstoff vorzugsweise zeitgleich oder gleichzeitig vorzunehmen.

DE 2934190 A1 offenbart ein Verfahren zur Molekülspektroskopie, insbesondere zur Bestimmung von Stoffwechselprodukten, bei dem die Absorption von Infrarotstrahlung durch eine Probe, welche eine zu bestimmende Substanz enthält, gemessen wird. Als am meisten bevorzugt zu bestimmende Substanz wird Glucose genannt. Dabei erfolgt die Glucosebestimmung im Vollblut oder Serum oder auch im Harn mit Raman- oder Kohlendioxid ($CO_2$)-Lasern als Lichtquelle. DE 2934190 A1 beschreibt, dass die Konzentration mehrerer Stoffe gemessen werden kann, wobei mehrere Wellenlängen simultan durch die Probe gestrahlt werden müssen. Außerdem weist DE 2934190 A1 auf die Möglichkeiten zur Entwicklung einer extrakorporalen oder auch implantierbaren künstlichen Bauchspeicheldrüse hin. Das in DE 2934190 A1 beschriebene Verfahren zeigt dem Fachmann, dass es grundsätzlich möglich ist, mittels Infrarotspektroskopie mehrere Stoffe gleichzeitig zu bestimmen, d. h. deren Anwesenheit und, angeblich, auch deren Konzentrationen. Dieses Verfahren ist jedoch ungeeignet, Blutproben oder Blutserumproben zu vermessen. Ferner weisen die im Blut oder Dialysatfluss zu bestimmenden Substanzen kein klar differenzierbares Infrarotspektrum aus. Die in DE 2934190 A1 vermessenen Substanzen sind gut dazu geeignet, mittels Infrarot bestimmt zu werden. Aufgrund der deutlich anderen Stoffeigenschaften, welche mittels Infrarot und UV bestimmt werden, lässt sich die in DE 2934190 A1 beschriebene Messanordnung und die Messverfahren nicht auf den UV-Bereich übertragen. Im UV-Bereich wird die mit der Konzentration eines Stoffes in Verbindung stehende Absorption des gesamten Moleküls bestimmt, während mittels IR bestimmte Arten von Bindungen in einem Molekül angeregt werden und somit IR vorrangig dazu dient, das Vorhandensein bestimmter funktioneller Gruppen nachzuweisen. Das in DE 2934190 A1 beschriebene IR-Meßgerät kann daher nicht einfach durch ein UV-Meßgerät oder durch ein NMR-Meßgerät ersetzt werden, weil es sich um grundlegend andere Techniken handelt, welche nicht äquivalent austauschbar sind.

DE 69916053 T2 bezieht sich auf ein Verfahren zum Bestimmen von Abfallprodukten in der Dialyseflüssigkeit bei Dialysebehandlungen. Das Verfahren dient zum genauen Bestimmen der Menge an Abfallprodukten in der Dialyseflüssigkeit während der Dialysebehandlung sowie der Messung von Harnstoff oder einer anderen in den Abfallprodukten enthaltenen Substanz. So kann die Bestimmung wahlweise durch Messen der Substanzen vorgenommen werden, die für die Auswahl des Dialysators und die Steuerung der Dialysemaschine am besten geeignet sind, um so die Dialysebehandlung an den Patienten anzupassen. DE 69916053 T2 gibt keinen Hinweis auf die Verwendung von Leuchtdioden und verzichtet auf eine Referenzsubstanz. Ferner gibt DE 69916053 T2 dem Fachmann keinen Hinweis, wie mehrere oder alle UV-aktiven Stoffe im Blut oder Dialysatabfluss quantitativ bestimmt werden können.

US 5772606 offenbart ein Urinal mit einem Meßsystem, mit dem die Mengen der Harnbestandteile Glucose, Hämoglobin, Albumin, Lithiumacetacetat, Ascorbinsäure, Kreatinin, Natriumchlorid und Natriumnitrit bestimmt werden können. Das von US 5772606 offenbarte Meßsystem nutzt den Wellenlängenbereich zwischen 400 und 2500 Nanometer. Als Lichtquelle werden Laser offenbart.

JP 02027264 A beschreibt die Messung von Proteinen im Urin bei einer Wellenlänge von 610 Nanometer. Als Lichtquelle werden Leuchtdioden verwendet. Proteine absorbieren jedoch auch im UV-Bereich unter 210 Nanometer und bei 280 Nanometer. Allerdings ist die Absorption bei 280 Nanometer vom Vorhandensein der Aminosäuren Tryptophan und Tyrosin in der Aminosäuresequenz nötig, da andere Aminosäuren nicht im UV-Bereich absorbieren, wobei Disulfidbrücken und Phenylalanin die UV-Absorption minimal beeinflussen. Unterhalb von 210 Nanometer absorbieren in einem Protein die Peptidbindungen. Aufgrund der Häufigkeit der Peptidbindungen in einem Protein, ist dies eine sehr sensitive Region des Proteinspektrums. Damit ist eine quantitative Proteinbestimmung in Probenflüssigkeiten zwar möglich, aber anhand des erzielten Spektrums können ohne Kenntnis des jeweiligen Extinktionskoeffizienten die enthaltenen Proteine nicht identifiziert werden. Auch ist mit der in JP 02027264 A offenbarten Vorrichtung und dem Verfahren keine Bestimmung von urämischen Toxinen möglich. Die Bestimmung im Infrarotbereich weist den Nachteil auf, dass sich die zu messende Probe durch die die Probe passierende Infrarotstrahlung erwärmt. Dies kann zur Umlagerung oder zum Abbau der zu bestimmenden urämischen Toxine und zu einer Änderung des Extinktionskoeffizienten führen, so dass mit der erfindungsgemäßen Vorrichtung zur extrakorporalen Blutbehandlung zur Unterscheidung urämischer Toxine im Abfluss der Vorrichtung die urämischen Toxine im Abfluss der Vorrichtung nicht mehr unterschieden werden können. Infrarotmessungen untersuchen andere Stoffeigenschaften als UV-Messungen. Die Messmethoden sind daher stoffspezifisch zu betrachten, da andere optische Eigenschaften benötigt werden. Daher ist es mit dem in JP 02027264 A beschriebenen Versuchsaufbau nicht möglich, quantitativ mehrere oder alle UV-aktiven Stoffe im Blut oder Dialysatabfluss zu bestimmen. Wie oben ausgeführt, sind IR-Spektroskopie und UV-Spektroskopie auf deutlich andere Moleküleigenschaften gerichtet und beide Methoden lassen sich nicht als äquivalent austauschen.

Bekannte Verfahren zur Protein- und Harnsäurebestimmung im Urin haben sich in der Praxis bewährt. Nachteilig ist jedoch, dass bisher keine Möglichkeit zur Unterscheidung urämischer Toxine in der verbrauchten Dialysierflüssigkeit bestand, und damit eine Überprüfung des Dialyseerfolgs des Dialysats der Dialysemaschine oder Teilen davon, die zur Blutreinigung beitragen, nicht möglich war.

Die vorliegende Erfindung ist auf die Bereitstellung einer Vorrichtung zur extrakorporalen Blutbehandlung zur quantitativen und vorzugsweise zur qualitativen und quantitativen Unterscheidung urämischer Toxine im Abfluß der Vorrichtung gerichtet.

Die Erfinder fanden überraschenderweise, dass entgegen der Annahme im Stand der Technik eine qualitative als auch quantitative Aussage über zusätzliche Abfallstoffe bzw. toxische Substanzen, die sich zumeist zusätzlich zu Harnstoff in der verbrauchten Dialysierflüssigkeit befinden, während bzw. zeitnah zur Therapie des Patienten möglich ist, falls bei mehreren Wellenlängen im UV-Bereich die Absorbanz der verbrauchten Dialysierflüssigkeit gemessen wird.

Zu den urämischen Toxinen zählen Substanzen, die unter normalen Bedingungen von einer gesunden Niere ausgeschieden werden, im Erkrankungsfall aber zurückbehalten werden. Die urämischen Toxine können biologische Funktionen negativ beeinflussen. Man unterscheidet freie, wasserlösliche Substanzen mit niedrigem Molekulargewicht (siehe Tabelle 1) von proteingebundenen Substanzen (siehe Tabelle 2).

**Tabelle 1: Freie wasserlösliche Stoffe im Blutplasma bzw. Blutserum.** Modifiziert nach Vanholder et al. *Review on uremic toxins: classification, concentration and interindividual variability.* Kidney International. 2003;63:1934-1943. * Absorption im UV-Bereich.

| | | |
|---|---|---|
| 1-Methyladenosin | Erythritol | Orotsäure |
| 1-Methylguanosin | Guanidin | Oxalate |
| 1-Methylinosin | Guanidinessigsäure | Phenylacetylglutamin |
| Asymmetrisches Dimethylarginin (ADMA) | Guanidonbernsteinsäure | Pseudouridin |

(fortgesetzt)

| α-Keto-δ-guanidinovaleriansäure | Harnsäure* | Symmetrisches Dimethylarginin (SDMA) |
|---|---|---|
| α-N-Acetylarginin | Harnstoff | Sorbitol |
| Arab(in)itol | Hypoxanthin | Taurocyamin |
| Argininsäure | Malondialdehyd* | Threitol |
| Benzylalkohol | Mannitol | Thymin |
| ß-Guanidinopropionsäure | Methylguanidin | Uracil |
| ß-Lipotropin | Myoinositol | Uridin |
| Creatin | $N^2,N^2$-Dimethylguanosin | Xanthin |
| Creatinin* | $N^4$-Acetylcytidin | Xanthosine |
| Cytidin | $N^6$-Methyladenosin | |
| Dimethylglycin | $N^6$-Threonylcarbamoyladenosin | |

Neben den freien wasserlöslichen Substanzen gibt es proteingebunden Substanzen, die in Tabelle 2 zusammengefasst sind.

**Tabelle 2: Proteingebundene Substanzen im Blutplasma bzw. Blutserum.** Modifiziert nach Vanholder et al. (2003). * Absorption im UV-Bereich.

| 2-Methoxyresorcinol | Indol-3-essigsäure | Pentosidin |
|---|---|---|
| 3-Deoxyglucoson | Indoxylsulfat* | Phenol* |
| 3-Carboxy-4-methyl-5-propyl-2-furanopropanonsäure (CMPF) | Kynurenin | P-OH-Hippursäure |
| Fructoselysin | Leptin | Putrescin |
| Glyoxal | Melatonin | Quinolinsäure |
| Hippursäure* | Methylglyoxal | Retinol-bindendes Protein* |
| Homocystein | $N^ε$-(carboxymethyl)lysin | Spermidin |
| Hydroquinon | *p*-Cresol* | Spermin |

Des Weiteren zählen Substanzen mit mittlerem Molekulargewicht zu den urämischen Toxinen im Blutplasma bzw. Blutserum, z. B. Adrenomedullin, atriales natriuretisches Peptid, Cystatin, Endothelin und Parathormon.
Tabelle 3 zeigt schließlich eine Übersicht urämischer Toxine, deren genaue Urinkonzentrationen und/oder deren Harnpflicht diskutiert werden und somit vorteilhafter Weise auch aus dem Blut entfernt werden sollten.

**Tabelle 3: Substanzen im Blutplasma bzw. Blutserum, deren Konzentration und/oder Harnpflicht nicht gesichert ist.** Modifiziert nach Vanholder et al. (2003). *Absorption im UV-Bereich.

| 1-Alkyl-2-formyl-3,4-glycosyl-pyrrol | $ß_2$-Microglobulin-Fragmente* | $N^ε$-Carboxyethyllysin |
|---|---|---|
| 2-(2-Fuoryl)-4(5)-(2-furanyl)-1H-imidazol | Cadaverin | Organische Chloramine |
| 3-Deoxyfructoson | Crosslin | Oxidiertes *low-density* Lipoprotein |
| 3-Hydroxykinurenin | Dimethylamin | Parathormonfragmente |
| 4-Hydroxynonenal* | Guanosin | Pyrralin |
| *Advanced oxidation protein products* (AOPP) | Imidazolone | Pyrrolaldehyd |

(fortgesetzt)

| Advanced glycation end products-ß₂-Microglobulin | Methylamin | Trimethylamin |
|---|---|---|
| Anthranilsäure | | |

Die mittels UV-Absorption bestimmbaren urämischen Toxine sind in den Tabellen 1 bis 3 durch das Zeichen "*" markiert. Als zu bestimmende urämische Toxine sind die UV-aktiven Substanzen Creatinin, Harnsäure, Hippursäure, Indoxylsulfat, 4-Hydroxynonenal, Malondialdehyd, *p*-Cresol, Phenol, Retinol-bindendes Protein und ß₂-Microglobulin-Fragmente und/oder Kombinationen davon bevorzugt. Mittels UV-Absorption bestimmbar bedeutet, dass das urämische Toxin im UV-Bereich absorbiert, d. h. das urämische Toxin ist UV-aktiv und kann daher auch als UV-aktives urämisches Toxin bezeichnet werden.

Es ist Aufgabe der Erfindung, eine Vorrichtung zur Verfügung zu stellen, welche die Bestimmung von mehr als einem UV-aktiven urämischen Toxin oder aller UV-aktiven urämischen Toxine während einer Dialysesitzung oder innerhalb kurzer Zeitabstände oder zeitgleich im Blut oder Dialysatabfluss zulässt, um daraus Rückschlüsse auf die Qualität der Dialyse als auch des Dialyseapparates oder bestimmter Bauteile des Dialyseapparates, welche die Abtrennung der urämischen Toxine mitbestimmen, zu ziehen. Die erfindungsgemäße Vorrichtung und das Verfahren lassen zum einen während der bzw. zeitnah zur Therapie des Patienten wenigstens eine qualitative als auch quantitative Aussage über einen weiteren Abfallstoff bzw. eine weitere toxische Substanz zu, die sich z.B. zusätzlich zu Harnstoff in der verbrauchten Dialysierflüssigkeit befindet. Des weiteren dient die erfindungsgemäße Vorrichtung und das Verfahren dazu, eine Aussage über die Qualität einer durchgeführten Dialyse zu treffen und damit z.B. auch über neue Membranen, Filter, Beschichtungen, Dialysierflüssigkeiten, Dialysatoren oder Dialyseapparate und deren eventuelle vorteilhafte Eigenschaften im Vergleich zu bekannten Ausführungsformen.

Der weitere Abfallstoff bzw. die weiteren toxischen Substanzen oder urämischen Toxine, die sich zusätzlich zu Harnsäure in der verbrauchten Dialysierflüssigkeit befinden, werden bevorzugt aus der Kreatinin, Malondialdehyd, Indoxylsulfat und p-Cresylsulfat bzw. p-Cresol umfassenden Gruppe ausgewählt. Auch Kombinationen der vorgenannten Substanzen sind möglich. Bevorzugt ist eine Kombination aus Harnsäure, Creatinin und Malondialdehyd. Noch bevorzugter ist eine Kombination aus Harnsäure, Creatinin, Malondialdehyd, Indoxylsulfat und p-Cresylsulfat. Am meisten bevorzugt ist eine Kombination der UV-aktiven urämischen Toxine umfassend Creatinin, Harnsäure, Hippursäure, Indoxylsulfat, 4-Hydroxynonenal, Malondialdehyd, *p*-Cresol, Phenol, Retinol-bindendes Protein und ß₂-Microglobulin-Fragmente.

Gelöst wird diese Aufgabe durch eine Vorrichtung mit den Merkmalen des Patentanspruchs 1. Vorteilhafte Ausgestaltungen der Erfindung finden sich in den Unteransprüchen, den Figuren, den Beispielen und der Beschreibung.

Die vorliegende Erfindung ist auf eine Vorrichtung zur extrakorporalen Blutbehandlung gerichtet, welche folgende Komponenten umfasst oder aus folgenden Komponenten besteht:

- einem Dialysator, der durch eine semipermeable Membran in eine erste und zweite Kammer (29, 30) geteilt ist, wobei die erste Kammer (29) in einem Dialysierflüssigkeitsweg angeordnet ist und die zweite Kammer (30) mittels einer Blutzuführleitung (32) und einer Blutabführleitung (31) mit dem Blutkreislauf eines Patienten verbindbar ist,
- einem Zulauf (20) für frische Dialysierflüssigkeit,
- einem Ablauf (30) für verbrauchte Dialysierflüssigkeit,
- einer in dem Ablauf (36) angeordneten Messeinrichtung (37), wobei die Messeinrichtung (37) eine Strahlungsquelle (1) für elektromagnetische UV-Strahlung aufweist,
- wobei die Strahlungsquelle (1) entweder aus mindestens zwei monochromatischen Lichtquellen oder mindestens einer polychromatischen Lichtquelle mit Monochromatoren zur Erzeugung monochromatischer UV-Strahlung besteht,
- einer Mikroprozessoreinheit (14), einer Speichereinheit sowie einer Ausgabeeinheit (15), dadurch gekennzeichnet, dass

die Messeinrichtung (37) dazu ausgebildet ist, im Wesentlichen monochromatische elektromagnetische UV-Strahlung unterschiedlicher Wellenlängen zu erzeugen und durch den Ablauf (36) für verbrauchte Dialysierflüssigkeit zu führen, wobei wenigstens ein Detektorsystem (5) zur Detektion der Intensität oder Absorbanz der durch den Ablauf (36) für verbrauchte Dialysierflüssigkeit hindurch tretenden im Wesentlichen monochromatischen elektromagnetischen UV-Strahlung vorgesehen ist und in der Speichereinheit ein Gleichungssystem $A_{\lambda i} = \sum_{j=1}^{n} A_j$ hinterlegt ist, wobei $A_{\lambda,i}$ die gesamte Absorbanz eines Stoffgemisches bei einer vorgegebenen Wellenlänge $\lambda_i$, $A_j$ die Absorbanz eines einzelnen Stoffes innerhalb des Stoffgemisches und *n* die Anzahl der interessierenden Komponenten innerhalb eines Stoffgemisches, welche zur Absorbanz beitragen, bezeichnen.

Werden mindestens 2, vorzugsweise 5, weiter bevorzugt 8, noch weiter bevorzugt 10, noch weiter bevorzugt 12, bevorzugt 14 und bevorzugt 16 monochromatische Lichtquellen wie z.B. LEDs verwendet, so sind diese vorzugsweise einzeln ansteuerbar.

Zur Detektion der Intensität oder Absorbanz bei einer bestimmten Wellenlänge wird nur eine Wellenlänge an einem Ort durch den Dialysatabfluß, d.h. die verbrauchte Dialysierflüssigkeit gestrahlt. Natürlich können erfindungsgemäß auch gleichzeitig mehrere Absorbanzmessungen vorgenommen werden, wobei dann an verschiedenen ausreichend voneinander getrennten Orten monochromatische Wellenlängen durch die verbrauchte Dialysierflüssigkeit geleitet werden.

Bevorzugt ist, dass an einer Stelle am Dialysatablauf zu einer gegebenen Zeit mittels einer Wellenlänge, d.h. einer monochromatischen UV-Strahlung oder einer polychromatischen Strahlung und entsprechender Filter oder Monochromatoren die Absorbanz der verbrauchten Dialysierflüssigkeit bei einer bekannten Temperatur und über eine bekannte Wegstreckt bestimmt wird, um daraus erfindungsgemäß die Konzentration der UV-aktiven urämischen Toxine in der verbrauchten Dialysierflüssigkeit zu bestimmen.

Ein weiteres Beispiel ist auf ein Verfahren zur Bestimmung der Konzentration von UV-aktiven urämischen Toxinen gerichtet, wobei im Dialysatablauf die Absorbanz der verbrauchten Dialysierflüssigkeit bei einer bekannten Temperatur und über eine bekannte Wegstreckt bei bestimmten Wellenlängen gemessen wird und mindestens so viele Messungen bei verschiedenen Wellenlängen durchgeführt werden wie UV-aktive urämische Toxine in der verbrauchten Dialysierflüssigkeit enthalten sind und mittels des Gleichungssystems $A_{\lambda i} = \sum_{j=1}^{n} A_j$ , wobei $A_{\lambda,i}$ die gesamte Absorbanz eines Stoffgemisches aus UV-aktiven urämischen Toxinen bei einer vorgegebenen Wellenlänge $\lambda_i$, $A_j$ die Absorbanz eines einzelnen UV-aktiven urämischen Toxins innerhalb des Stoffgemisches und *n* die Anzahl der interessierenden Komponenten innerhalb eines Stoffgemisches, welche zur Absorbanz beitragen, bezeichnen und über diese Gleichung nach der Detektion der Absorbanz bei *n* charakteristischen Messpunkten die Konzentration der *n* UV-aktiven urämischen Toxine in dem Stoffgemisch bestimmt wird.

Mit der erfindungsgemäßen Vorrichtung ist es möglich, eine Differenzierung von vorzugsweise Harnsäure von anderen toxischen Substanzen bzw. Abfallprodukten im Dialysat dialysepflichtiger Patienten vorzunehmen, wobei die Vorrichtung von der optischen UV-Spektroskopie Gebrauch macht und mittels welcher alternierende oder gleichzeitige Messungen der Absorbanz des gebrauchten Dialysates bei verschiedenen im Wesentlichen monochromatischen Wellenlängen möglich sind. Die erfindungsgemäße Vorrichtung erlaubt vor allem die Bestimmung der Konzentration von allen im Blut oder im gebrauchten Dialysat, d.h. im Dialysatabfluß vorkommenden UV-aktiven urämischen Toxine. Im Stand der Technik ist noch keine Dialysemaschine offenbart, welche sämtliche UV-aktiven urämischen Toxine, nämlich Creatinin, Harnsäure, Hippursäure, Indoxylsulfat, 4-Hydroxynonenal, Malondialdehyd, p-Cresol, Phenol, Retinol-bindendes Protein und ß2-Microglobulin-Fragmente, während einer Dialysesitzung oder innerhalb von 30 Minuten, vorzugsweise innerhalb von 20 Minuten, weiter bevorzugt innerhalb von 15 Minuten, noch weiter bevorzugt innerhalb von 10 Minuten, noch weiter bevorzugt innerhalb von 8 Minuten, noch weiter bevorzugt innerhalb von 7 Minuten, noch weiter bevorzugt innerhalb von 6 Minuten und am meisten bevorzugt innerhalb von 5 Minuten quantitativ zu bestimmen vermag. Die UV-Messung kann dabei gleichzeitig oder zeitgleich erfolgen, d.h. dass bei mindestens 10 charakteristischen (Definition von "charakteristisch" s.u.) Wellenlängen oder Messpunkten gleichzeitig oder nacheinander bzw. unmittelbar nacheinander gemessen wird.

Die Vermessung von den bisher bekannten 10 UV-aktiven urämischen Toxinen verlangt hingegen nicht, dass keine anderen UV-aktiven Stoffe anwesend sein dürfen. Ganz im Gegenteil funktioniert das Verfahren weiterhin einwandfrei, falls weitere UV-aktive Stoffe im Blut oder Dialysatabfluß anwesend sein sollten, wie beispielsweise ein Medikament, welches UV-aktiv ist oder einen bestimmten UV-aktiven Nahrungsbestandteil, der vom Patienten durch Verzehr einer größeren Menge eines bestimmten Lebensmittels im Blut oder Dialysatabfluß nachweisbar ist. Solange pro UV-aktivem urämischen Toxin mindestens eine UV-Messung bei einem geeigneten Messpunkt durchgeführt wird, können alle 10 bisher bekannten UV-aktiven urämischen Toxine quantitativ oder quantitativ und qualitativ bestimmt werden. Wie die Auswahl geeigneter Messpunkt erfolgt, wird weiter unten eingehend beschrieben. Die UV-Messung kann im Blut oder im Dialysatabfluß durchgeführt werden, wobei im Dialysatabfluß genauer als im Blut gemessen werden kann, d.h. die Konzentrationsbestimmung im Dialysatabfluß genauer erfolgen kann, wobei dann zu berücksichtigen ist, dass die Konzentration der gemessenen Substanz im Dialysatabfluß nicht zwingend identisch mit der Konzentration dieser Substanz im Blut sein muss, sofern nicht dafür gesorgt wird, dass sich z.B. durch einen zeitweise geschlossenen Kreislauf, wo der Dialysatabfluß wieder in den Dialysator eingeführt wird, sich die Konzentrationen der Stoffe im Dialysatabfluß den Konzentrationen im Blut angleichen können.

Der Begriff "Dialysatabfluß" wie hierin verwendet, bezeichnet das verbrauchte Dialysat, welches nach dem Passieren des Dialysators als Abfallprodukt aus dem Dialysator austritt. Wie hierin verwendet bezeichnet der Begriff "Toxine" oder "urämische Toxine" die hierin offenbarten UV-aktiven urämischen Toxine, nämlich Creatinin, Harnsäure, Hippursäure, Indoxylsulfat, 4-Hydroxynonenal, Malondialdehyd, p-Cresol, Phenol, Retinol-bindendes Protein und ß2-Microglobulin-Fragmente.

Humanes Beta-2-Microglobulin ist ein Serumprotein, das als Volllängenprotein aus einer singulären Polypeptidkette aus 119 Aminosäuren besteht (GenBANK-Zugangsnummer CAG33347, Version CAG33347.1, GI:48146249 vom 17. April 2005). Das Molekulargewicht von humanem Beta-2-Microglobulin in Volllänge beträgt 11,6 kD. Fragmente von humanen Beta-2-Microglobulin können durch enzymatischen Abbau des humanen Beta-2-Microglobulin entstehen, wobei mindestens zwei Fragmente des humanen Beta-2-Microglobulins entstehen. Jedes des durch den enzymatischen Abbau entstehenden Beta-2-Microglobulin-Fragments besteht aus einer Polypeptidkette, die weniger als 119 Aminosäuren des humanen Beta-2-Microglobulins in Volllänge umfasst.

Für möglichst genaue Messungen durchzuführen, erfolgt die UV-Messung bei im Wesentlichen monochromatischen Wellenlängen oder einfacher ausgedrückt, bei monochromatischen Wellenlängen. Im Wesentlichen monochromatische Wellenlängen sind durch eine Spitzenwellenlänge, durch eine dominante Wellenlänge und eine Schwerpunktswellenlänge gekennzeichnet, wobei die Spitzenhalbwertsbreite im Bereich zwischen 10 und 25 Nanometer liegt. Eine Abnahme der Spitzenhalbwertsbreite führt also zu einem engeren Wellenlängenbereich für die Spitzenwellenlänge. Allgemein versteht man unter der Halbwertsbreite einer Funktion mit einem Maximum die Differenz zwischen den beiden Argumentwerten, für die die Funktionswerte auf die Hälfte des Maximums abgesunken sind. Als Leuchtdioden können beispielsweise Leuchtdioden von Hamamatsu oder LaserComponents oder Leuchtdioden anderer Hersteller mit äquivalenten Spezifikationen verwendet werden.

Absorbanzmessungen im gebrauchten Dialysat bei einer Wellenlänge von λ=280 nm wurde bereits durch EP 1 083 948 B1 beschrieben. Die vorliegende Erfindung macht grundsätzlich von dem in EP 1 083 948 B1 offenbarten Sensor Gebrauch. Die Neuerung der Erfindung besteht darin, dass durch die Verwendung von mehreren einzelnen im Wesentlichen monochromatischen Wellenlängen Absorbanzmessungen durchgeführt werden können. Die dabei verwendeten Wellenlängen sind dadurch charakteristisch, dass im gebrauchten Dialysat, d.h. im Dialysatabfluß vorzugsweise nur die zu bestimmenden Abfallstoffe bei diesen Wellenlängen zur Absorbanz beitragen, wobei diese sich bei der gemessenen Wellenlänge überlagern dürfen und sogar auch mit anderen nicht zu bestimmenden UV-aktiven Stoffen überlagern können. Zur Bestimmung der Konzentration der UV-aktiven urämischen Toxine ist es zwingend, dass Wellenlängen verwendet werden, die charakteristische Punkte des verbrauchten Dialysats darstellen. Unter einem charakteristischen Meßpunkt des verbrauchten Dialysats ist zu verstehen, dass an diesem Punkt des Spektrums, d. h. bei dieser Wellenlänge, die Absorbanz im Wesentlichen von der zu bestimmenden Substanz oder mehreren zu bestimmenden Substanzen bestimmt wird. Ein charakteristischer Punkt des Spektrums des verbrauchten Dialysats kann von beispielsweise lokalen Maxima, lokalen Minima und/oder Wendepunkten unabhängig sein, wobei bei dem charakteristischen Wellenlängen oder Messpunkten entweder nur ein urämisches Toxin die Absorption bestimmen sollte oder bei der Überlagerung mehrerer UV-aktiver urämischer Toxine jedes Toxin deutlich zur Gesamtabsorption bei dieser Wellenlänge beitragen sollte, so dass der Anteil eines jeden Toxins an der Gesamtabsorption bei dieser Wellenlänge möglichst genau bestimmt werden kann, wobei gemäß der hierin offenbarten Gleichungssysteme auf die Konzentration zurückgerechnet werden kann. Zudem wird vorteilhafter Weise bei charakteristischen Punkten, d. h. Wellenlängen, gemessen, bei denen sich maximal zwei oder drei oder vier Stoffe überlagern, d. h. dort absorbieren, wobei die Zahl der charakteristischen Punkte, d. h. Wellenlängen, mindestens der Zahl zu bestimmender Toxine entspricht, da sonst das Gleichungssystem nicht gelöst werden kann. Als charakteristische Wellenlängen oder Messpunkte sind auch Flankenpunkte geeignet, welche zwischen einem Maxima und einem Wendepunkt liegen, sofern dort die überlagernden Toxine deutlich zur Absorption beitagen. Als ungeeignet können Messpunkte bzw. Wellenlängen für die Messung bezeichnet werden, wo beispielsweise 8 Toxine überlagern und nur 2 Toxine mit jeweils 40% die Gesamtabsorption bestimmen und die restlichen 20% zu weitgehend gleichen Anteilen von den restlichen 6 Toxinen bestimmt werden. Jedes einzelne der 6 restlichen Toxine kann in Bezug auf die Gesamtabsorption vernachlässigt werden, wobei jedoch die Vernachlässigung aller 6 restlichen Toxine zu einem zu großen Fehler in Bezug auf die beiden dominierenden Toxine führen würde und gleichzeitig die 6 restlichen Toxine einzeln nur so wenig zur Gesamtabsorption betragen, dass deren Konzentrationsbestimmung an diesem Messpunkt ebenfalls mit einem zu großen Fehler behaftet ist. Derartige Punkte werden als ungeeignet oder als nicht charakteristisch bezeichnet und sollten vermieden werden. Ferner sei hier erwähnt, dass das Verfahren und die erfindungsgemäße Vorrichtung nicht auf die bisher bekannten maximal 10 urämischen Toxine beschränkt ist, sondern grundsätzlich eine beliebige Anzahl beispielsweise weitere 5, oder weitere 10 oder weitere 15 oder weitere 20 UV-aktive Stoffe zusammen mit den 10 bekannten urämischen Toxinen quantitativ bestimmt werden können, sofern die UV-Spektren der Einzelverbindungen der weiteren UV-aktiven Stoffe und deren Extinktionskoeffizienten bekannt sind.

Eine solche erfindungsgemäße Vorrichtung kann durch den Gebrauch mehrerer Lichtquellen mit monochromatischer Strahlung oder einer polychromatischen Lichtquelle mit einem steuerbaren wellenlängenselektivem Element realisiert werden, wobei die Bestimmung der Absorbanz von urämischen Toxinen bei unterschiedlichen Wellenlängen erfolgt und mit Hilfe von wellenlängenabhängigen Extinktionskoeffizienten $\varepsilon_{\lambda i}$ die Konzentrationen urämischer Toxine berechnet werden. Grundsätzlich wird immer die Absorbanz des verbrauchten Dialysats gemessen, die sich aus Summe der Absorbanz jedes einzelnen urämischen Toxins zusammensetzt. Dabei reicht es allerdings nicht aus, die Absorbanz jedes einzelnen urämischen Toxins zu kennen. Um eine Konzentrationsbestimmung durchzuführen, müssen der stoffspezifische Extinktionskoeffizient und die optische Messstrecke bekannt sein (Gleichung 1). Jede zu bestimmende UV-aktive Substanz oder UV-aktives urämisches Toxin ergibt sich als eine Unbekannte einer Gleichung, so dass pro UV-aktive Substanz zumindest eine UV-Messung durchgeführt werden muß. Bei einer zu bestimmenden UV-aktiven Substanz oder UV-aktives urämisches Toxin ergibt sich also eine Gleichung mit einer Unbekannten, so dass die Messung bei mindestens einer Wellenlänge im UV-Bereich durchgeführt werden muß. Bei zwei zu bestimmenden UV-aktiven Substanzen ergibt sich eine Gleichung mit zwei Unbekannten, so dass die Messung bei mindestens zwei Wellenlängen im UV-Bereich durchgeführt werden muß. Bei drei zu bestimmenden UV-aktiven Substanzen ergibt sich eine Gleichung mit drei Unbekannten und einer Messung bei mindestens drei Wellenlängen usw.. Allgemein ergibt sich bei $n$ zu bestimmenden UV-aktiven Substanzen eine Gleichung mit $n$ Unbekannten, so dass die Messung bei mindestens $n$ Wellenlängen im UV-Bereich durchgeführt werden muß.

Unter monochromatischer elektromagnetischer Strahlung versteht man Strahlung mit einer definierten Wellenlänge. Monochromatische elektromagnetische Strahlung kann von einer Leuchtdiode erzeugt werden. Leuchtdioden sind elektronische Halbleiterelemente, die bei Stromdurchfluss in Durchlassrichtung elektromagnetische Strahlung in einem begrenzten Spektralbereich emittieren. Die emittierte elektromagnetische Strahlung ist nahezu monochrom. Die Wellenlänge kann, je nach Ausgestaltung der Leuchtdiode, im sichtbaren Bereich des Spektrums, im Infrarotbereich oder im Ultraviolettbereich liegen. Die Strahlungsquelle für die erfindungsgemäße Vorrichtung bestehend aus mehreren Lichtquellen zur Emission monochromatischer elektromagnetischer Strahlung sollte zur Emission elektromagnetischer Strahlung im Bereich von 1 Nanometer bis 750 Nanometer ausgebildet sein. Insbesondere muß die Strahlungsquelle bestehend aus mehreren Lichtquellen zur Emission elektromagnetischer Strahlung im Bereich der ultravioletten Strahlung von 180 Nanometer bis 380 Nanometer ausgebildet sein. Für die Messung von urämischen Toxinen sind Leuchtdioden bevorzugt, die im Bereich der ultravioletten Strahlung von 180 Nanometer bis 380 Nanometer, weiter bevorzugt im Bereich der ultravioletten Strahlung von 180 Nanometer bis 320 Nanometer emittieren. Um alle UV-aktiven Substanzen detektieren zu können, müssen die Detektoren ausreichend sensitiv sein, da sich einige Substanzen nur sehr geringfügig voneinander unterscheiden lasen, was das UV-Spektrum angeht. Außerdem muss die Auflösung der Messeinrichtung hinreichend groß sein.

Bei der Verwendung von Leuchtdioden ist weiterhin vorteilhaft, dass Leuchtdioden keine thermischen Strahler sind und die bei der Strahlungserzeugung entstehende Wärme z. B. durch Kühlrippen auf der Rückseite der Leuchtdioden abgeführt werden kann.

Alternativ kann die Strahlungsquelle bestehend aus mehreren Lichtquellen zur Erzeugung polychromatischer elektromagnetischer Strahlung ausgebildet sein. Um die im Wesentlichen monochromatische elektromagnetische Strahlung im Bereich von 1 Nanometer bis 750 Nanometer, bevorzugt im Bereich von 170 Nanometer bis 380 Nanometer und weiter bevorzugt 180 nm bis 320 nm, zu erzeugen, sind entsprechende Monochromatoren vorgesehen. Insbesondere sind nur für eine spezifische Wellenlänge durchlässige optische Filter bzw. ein Bandpassfilter mit mehreren Passbereichen vorgesehen. Strahlungsquelle bestehend aus mehreren Lichtquellen zur Erzeugung polychromatischer elektromagnetischer Strahlung im ultravioletten Bereich des Spektrums sind zum Beispiel Quecksilberdampflampen oder Deuteriumlampen. Für die vorliegende Erfindung können herkömmliche Quecksilberdampflampen und/oder Deuteriumlampen, optische Filter und Bandpassfilter mit mehreren Passbereichen verwendet werden. Die Anpassung der herkömmlichen Quecksilberdampflampen und/oder Deuteriumlampen, optische Filter und Bandpassfilter mit mehreren Passbereichen zur Erzeugung monochromatischer elektromagnetischer Strahlung im Bereich von 1 Nanometer bis 750 Nanometer, bevorzugt im Bereich von 180 Nanometer bis 380 Nanometer und weiter bevorzugt 190 nm bis 320 Nanometer an das Absorptionsverhalten der zu bestimmenden urämischen Toxine liegt im Können eines Fachmanns.

Im Folgenden werden die theoretischen Grundlagen näher beschrieben.

Die Absorbanz ergibt sich nach dem Lambert-Beerschen Gesetzt wie folgt:

$$A_{\lambda_i} = \varepsilon_{\lambda_i} \cdot l \cdot c \quad (1)$$

wobei $A_{\lambda i}$ die Absorbanz bei einer bestimmten Wellenlänge $\lambda_i$, $\varepsilon_{\lambda i}$ den wellenlängenabhängigen Extinktionskoeffizienten, l die optische Weglänge und c die jeweilige Konzentration eines Stoffes beschreibt. Häufig wird auch vom Absorptionsquerschnitt gesprochen. Dieser drückt nach Gleichung (1) die auf die Länge normierten Absorbanz $\frac{A_{\lambda_i}}{l} = \varepsilon_{\lambda_i} \cdot c$ aus.

Besteht ein Stoffgemisch aus mehreren absorbierenden Stoffen, so setzt sich die Absorbanz des Stoffgemisches additiv aus den Absorbanzen der einzelnen Komponenten für eine konstante Wellenlänge $\lambda_i$ zusammen:

$$A_{\lambda i} = \sum_{j=1}^{n} A_j \quad (2)$$

In Gleichung (2) bezeichnet $A_{\lambda i}$ die gesamte Absorbanz eines Stoffgemisches, $A_j$ die Absorbanz eines einzelnen Stoffes innerhalb des Stoffgemisches und $n$ die Anzahl der Komponenten innerhalb eines Stoffgemisches, welche zur Absorbanz beitragen. j stellt dabei den Laufindex der mathematischen Summenoperation dar. Die Formeln (1) und (2) gelten nur, solange die Wellenlänge $\lambda_i$ konstant ist. Mit diesem System kann das Absorptionsspektrum von verbrauchtem Dialysat in ein Gleichungssystem von $n$ Gleichungen bei $n$ verschiedenen Wellenlängen zerlegt werden, um daraus die Konzentration von $n$ urämischen Toxinen zu identifizieren. Dies gelingt jedoch nur, falls die Spektren der urämischen Toxine bekannt sind und sich die Spektren der urämischen Toxine ausreichend unterscheiden bzw. die vorgenannten charakteristischen Meßpunkte existieren, was bei den UV-aktiven urämischen Toxinen der Fall ist.

Auswahlkriterien für einen charakteristischen Messpunkt bzw. eine charakteristische Wellenlänge:

Unter einem charakteristischen Punkt des verbrauchten Dialysats ist zum einen zu verstehen, dass an diesem Punkt des Spektrums, d. h. bei dieser Wellenlänge, die Absorbanz im Wesentlichen von der zu bestimmenden Substanz bestimmt wird. D.h. vorzugsweise wird die Absorbanz mindestens zu 90%, vorzugsweise mindestens 94% und insbesondere bevorzugt mindestens zu 96% von diesem einen Toxin bestimmt. Ein charakteristischer Messpunkt kann ferner dort liegen, wo im UV-Spektrum des reinen Toxins ein Maximum, Wendepunkt oder Flankenpunkt zwischen einem Maximum und einem Wendepunkt vorliegt. Darüber hinaus können charakteristische Messpunkte bzw. charakteristische Wellenlängen bei einem lokalen Maximum, lokalen Minimum, Flankenpunkten und/oder Wendepunkten des Gesamtspektrums der vermessenen Probe liegen. Ein charakteristischer Meßpunkt, d. h. eine Wellenlänge, kann beispielsweise anhand lokaler Maxima, lokaler Minima und/oder Wendepunkten der Spektren der urämischen Toxine gewählt werden, sofern diese sich zwischen den zu bestimmenden urämischen Toxinen unterscheiden sollten. Für den Fall, dass sich die lokalen Maxima, lokalen Minima und/oder Wendepunkte der Spektren der urämischen Toxine bei einer bestimmten Wellenlänge überlagern sollten, kann ein charakteristischer Meßpunkt in anderen Bereichen der Spektren der urämischen Toxine wie zum Beispiel in Bereichen der Spektren der urämischen Toxine mit positiver oder negativer Steigung gewählt werden. Für den Fall, dass die zu bestimmende Substanz an einem charakteristischen Punkt des Spektrums der Messlösung, d. h. einer bestimmten Wellenlänge absorbiert, trägt die Absorbanz zum Gleichungssystem bei. Für den Fall, dass an einem charakteristischen Punkt des Spektrums der Messlösung, d. h. einer bestimmten Wellenlänge, keine der zu bestimmenden Substanzen zur Absorption beiträgt, dann trägt die Absorbanz an diesem charakteristischen Punkt, d. h. dieser Wellenlänge, nicht zur Lösung des Gleichungssystems bei. Bevorzugt ist, dass an den charakteristischen Punkten des Spektrums der Messlösung wie z.B. dem Blut oder dem Dialysatabfluß mindestens eine zu bestimmende Substanz absorbiert. Es ist auch möglich, dass an charakteristischen Punkten, d. h. Wellenlängen gemessen wird, bei denen sich maximal zwei oder drei oder vier Stoffe überlagern, d. h. dort absorbieren, wobei die Zahl der charakteristischen Punkte, d. h. Wellenlängen, mindestens die Zahl der zu bestimmenden Stoffe beträgt, da sonst das Gleichungssystem nicht gelöst werden kann. Charakteristische Messpunkte oder charakteristische Wellenlängen für die UV-Messung sind somit solche Messpunkte und Wellenlängen, wo entweder nur ein Toxin die Absorption bestimmt oder wo sich zwei oder mehrere Toxine überlagern und die sich überlagernden Toxine einzeln deutlich zur Gesamtabsorption bei dieser Wellenlänge beitragen, so dass der Einzelbeitrag eines jeden Toxins bei der gemessenen Wellenlänge relativ genau bestimmt und daraus die Konzentration berechnet werden kann. Ungünstig sind hingegen Messpunkte und Wellenlängen, bei denen mehrere UV-aktive Stoffe überlagern, jedoch keiner dieser Stoffe die Absorption dominiert und alle UV-aktiven Stoffe im einzelnen nur wenig zur Gesamtabsorption beitragen. Somit ist eine Wellenlänge für die UV-Messung durchaus geeignet, auch wenn dort 10 UV-aktive Stoffe sich überlagern, sofern z.B. ein Stoff zu 50% die Gesamtabsorption bestimmt ein zweiter Stoff zu 40% die Gesamtabsorption bestimmt und die restlichen 8 Stoffe nur zu 10 % die Gesamtabsorption bestimmen, weil dann diese 8 Stoffe vernachlässigt werden können und die beiden dominierenden Stoffe dennoch relativ genau vermessen werden können.
Ferner ist essentiell für die Auswahl der charakteristischen Messpunkte und der charakteristischen Wellenlängen, dass bei n zu bestimmenden Konzentrationen und n Messpunkten keine redundanten Informationen erhalten werden dürfen, d.h. die UV-Messung bei einer der n Wellenlängen nicht zu einem Ergebnis oder einer Informationen führen darf, welche bereits bei einer anderen der n Messungen erhalten wurde. Dies ist beispielsweise der Fall, fall bei zwei Wellenlängen die Absorbanz von demselben Toxin maßgeblich bestimmt wird. D.h. bei beiden Wellenlängen lässt sich die Konzentration desselben Toxins bestimmen und man erhält eine redundante Information, weil zwei Messpunkte für die Bestimmung eines Toxins benötigt wurden. Derartige redundante Informationen sind vorteilhaft, um die Genauigkeit des Verfahrens zu erhöhen, verlangen jedoch, dass bei n Toxinen mehr als n Messungen durchgeführt werden, weil sich die redundanten Informationen erst bei einer (n+1)-Messung ergeben dürfen. Hat man n Toxine und n Messungen mit zwei redundanten Informationen, so führt dies dazu, dann nur die Konzentrationen von n-2 Toxinen bestimmt werden können. Zu Bestimmung der Konzentrationen aller n Toxine werden in diesem Fall mindestens noch 2 weitere Messungen benötigt.

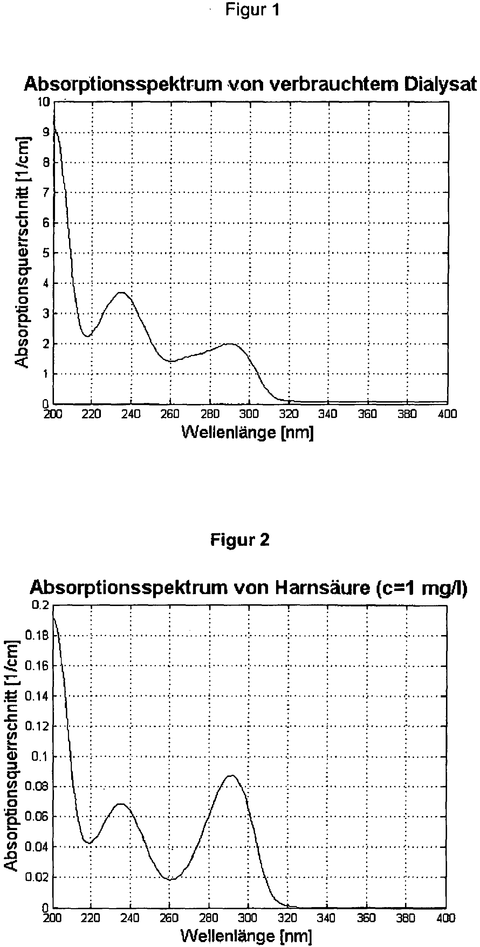

Geht man davon aus, dass das Absorbanzspektrum von Dialysepatienten bei einer bestimmten Wellenlänge im Wesentlichen von Harnsäure erzeugt wird, so vereinfacht sich das Gleichungssystem und man kann durch die Absorbanzmessung bei anderen Wellenlängen auf weitere UV-aktive urämische Toxine (siehe Tabellen 1 bis 3) zurückrechnen. Verdeutlicht wird dies durch die Abbildungen der Figuren 1 und 2. Figur 1 zeigt das typische Dialysatspektrum eines dialysepflichtigen Patienten, während Figur 2 den Verlauf des Harnsäurespektrums bei einer Konzentration von 1 mg/l zeigt. Deutlich ist zu erkennen, dass das Dialysatspektrum im Bereich von 320 nm bis 290 nm im Wesentlichen dem Spektrum von Harnsäure folgt. Im Wellenlängenbereich unter 290 nm unterscheiden sich die Spektren hingegen z. T. deutlich voneinander, so dass davon auszugehen ist, dass dort außer Harnsäure noch weitere Stoffe des Dialysatstoffgemisches an der Absorbanz beteiligt sind, während ab ca. 290 nm nur noch Harnsäure zur Absorbanz beiträgt.

Durch die Gleichungen (1) und (2) können für verschiedene Wellenlängen Gleichungssysteme aufgestellt werden, welche die Absorbanz eines Stoffgemisches für unterschiedliche Wellenlängen beschreiben. In Verbindung mit der Berechnung der Harnsäurekonzentration, welche bei $\lambda=290$ nm im Wesentlichen die Absorption im gesamten Dialysat ausmacht, kann durch die Bestimmung der Absorption bei z.B. $\lambda=266$ nm eine weitere toxische Substanz wie z.B. Malondialdehyd bestimmt werden, sofern die Absorption bei der zweiten Wellenlänge im wesentlichen von Harnsäure und eines zweiten Stoffes abhängt. Ähnlich wie am Beispiel von Malondialdehyd können auch andere Stoffe wie z.B. Creatinin, welche die Absorption des Dialysates im Wesentlichen mitbestimmen, durch dieses Verfahren bestimmt werden. Da Creatinin sein Absorptionsmaximum bei ca. $\lambda=235$ nm aufweist, ist es hier sinnvoll, die Absorbanz des Dialysates bei $\lambda=235$ nm zu bestimmen. Ebenfalls können auch weitere Stoffe, die in den Tabellen 1 bis 3 genannt sind und die Absorption des Dialysates im Wesentlichen mitbestimmen, durch dieses Verfahren bestimmt werden.

Aus dem Spektrum von Harnsäure gemäß Figur 2 lässt sich das Verhältnis der Absorbanz von Harnsäure bei zwei vorgegebenen Wellenlängen bestimmen. Dieses Verhältnis der Harnsäureabsorbanz gilt dann auch für die Absorbanz von Harnsäure im gebrauchten Dialysat. Infolgedessen lässt sich ein lineares Gleichungssystem mit mindestens zwei Komponenten aufstellen, und es ist möglich, die Konzentration bzw. die Reduktion von anderen urämischen Toxinen außer bzw. neben Harnsäure zu bestimmen, wie z. B. Malondialdehyd. Da davon auszugehen ist, dass Harnsäure fast immer im Dialysatabfluß anwesend sein wird, ist daher eine Messung bei $\lambda=290$ nm fast immer ratsam.

Folgende Gleichungen beschreiben die notwendigen Berechnungen beispielhaft für die UV-aktiven urämischen Toxine Harnsäure und Malondialdehyd:

$$A_{\lambda=290nm} \approx \varepsilon_{Harnsäue,\lambda_{290nm}} \cdot l \cdot c_{Harnsäure} \quad (3)$$

wobei $A_{\lambda=290\,nm}(Dialysat) = A_{\lambda=290\,nm}(Harnsäure)$ gilt.

Mit Gleichung 1 gilt weiter

$$A_{\lambda=290nm}(Harnsäure) = konst \cdot A_{\lambda=266nm}(Harnsäure) \quad (3a),$$

wobei *konst* das Verhältnis der Absorbanz von Harnsäure bei den Wellenlängen 290 nm und 266 nm ist.

Wird nun die Absorbanz bei einer Wellenlänge von 266 nm gemessen, bei der sich die Absorbanz im Wesentlichen nur aus den beiden Stoffen Harnsäure und Malondialdehyd zusammensetzt, so ergibt sich die Absorbanz des Dialysates bei 266 nm zu:

$$A_{\lambda=266nm}(Dialysat) = A_{\lambda=266nm}(Harnsäure) + A_{\lambda=266nm}(Malondialdehyd)$$

bzw. verallgemeinert zu:

$$A_{\lambda=266nm}(Dialysat) = A_{\lambda=266nm}(Harnsäure) + A_{\lambda=266nm}(Substanz\_x) \quad (3b)$$

Mit Lambert-Beer folgt wiederum:

$$A_{\lambda=266nm}(Dialysat) \approx \varepsilon_{Harnsäure,\lambda_{260nm}} \cdot l \cdot c_{Harnsäure} + \varepsilon_{Substanz\_x,\lambda_{260nm}} \cdot l \cdot c_{Substanz\_x} \quad (4)$$

Setzt man nun Gleichung 3a in Gleichung 4 ein, so folgt:

$$A_{\lambda=266nm}(Dialysat) = \frac{A_{\lambda=290nm}(Harnsäure)}{konst} + A_{Substanz\_x,\,\lambda_{266nm}},$$

$$= \frac{A_{\lambda=290nm}(Harnsäure)}{konst} + \varepsilon_{Substanz\_x,\lambda_{266nm}} \cdot l \cdot c_{Substanz\_x} \quad (4a)$$

und somit

$$c_{Substanz\_x} = \frac{A_{\lambda=290nm}(Harnsäure)}{konst \cdot l \cdot \varepsilon_{Substanz\_x,\lambda_{266nm}}} \quad (4b)$$

Ist die *Substanz_x,* welche beispielsweise neben Harnsäure den Hauptanteil an der Absorption bei $\lambda$=266 nm ausmacht bekannt, so kann daraus mit Hilfe von Gleichung 4a erst die Absorbanz dieser *Substanz_x* bei 266 nm bestimmt werden und nachfolgend mit Gleichung 4b die Konzentration der *Substanz_x.* Grundlage dafür ist, dass der zugehörige Extinktionskoeffizient $\varepsilon_{Substanz\_x,\lambda_{260nm}}$, welcher eine Stoffkonstante ist und daher im Labor ermittelt werden kann, bekannt ist.

Dieses Beispiel kann auch für mehrere unbekannte Stoffkonzentrationen herangezogen werden, sofern die Messung der Absorbanz mit mehreren Wellenlängen erfolgt. Allgemein spricht man hier von einem linearen Gleichungssystem beliebiger Ordnung. Nachfolgend ist als eine der zu bestimmenden Substanzen die Referenzsubstanz mit einer bevorzugten Wellenlänge $\lambda_1$ = 290 nm angegeben:

$$A_{\lambda i}(Dialysat) \approx \frac{A_{\lambda 1}(Referenzsubstanz)}{konst_i} + \sum_{j=2}^{n} \varepsilon_{j,\lambda_i} \cdot l \cdot c_j = \frac{A_{\lambda 1}(Referenzsubstanz)}{konst_i} + \sum_{j=2}^{n} A_j (4b)$$

wobei $\lambda_1$ die Wellenlänge ist, bei der die Referenzsubstanz im Wesentlichen die einzige Substanz im gebrauchten Dialysat ist, welche zur Absorbanz beiträgt, also bevorzugt 290 nm, und

$$konst_i = \frac{A_{\lambda 1}(Referenzsubstanz)}{A_{\lambda i}(Referenzsubstanz)} = \frac{\varepsilon_{\lambda 1}}{\varepsilon_{\lambda i}}$$ sowie j die betreffende Substanz, $c_j$ die entsprechende Konzentration der Substanz j im Dialysat und $A_j$ die Absorbanz der Substanz j bei der Wellenlänge $\lambda_i$.

In folgendem Beispiel ist vorzugsweise Harnsäure als eine der zu bestimmenden Substanzen angegeben:

$$A_{\lambda i}(Dialysat) \approx \frac{A_{\lambda 1}(Harnsäure)}{konst_i} + \sum_{j=2}^{n} \varepsilon_{j,\lambda_i} \cdot l \cdot c_j = \frac{A_{\lambda 1}(Harnsäure)}{konst_i} + \sum_{j=2}^{n} A_j (4c)$$

wobei $\lambda_1$ die Wellenlänge ist, bei der Harnsäure im wesentlichen die einzige Substanz im gebrauchten Dialysat ist, welche zur Absorbanz beiträgt, also bevorzugt 290 nm, und $konst_i = \frac{A_{\lambda 1}(Harnsäure)}{A_{\lambda i}(Harnsäure)} = \frac{\varepsilon_{\lambda 1}}{\varepsilon_{\lambda i}}$ sowie j die betreffende Substanz, $c_j$ die entsprechende Konzentration der Substanz j im Dialysat und $A_j$ die Absorbanz der Substanz j bei der Wellenlänge $\lambda_i$.

Die Referenzsubstanz wird aus der Gruppe umfassend die UV-aktiven urämischen Toxine Creatinin, Harnsäure, Hippursäure, Indoxylsulfat, 4-Hydroxynonenal, Malondialdehyd, p-Cresol, Phenol, Retinol-bindendes Protein und ß$_2$-Microglobulin-Fragmente und/oder Kombinationen davon ausgewählt. Als Referenzsubstanz ist Harnsäure bevorzugt.

Durch die Verwendung von Harnsäure als Referenzsubstanz kann das Gleichungssystem (4b) vereinfacht werden. Das oben beschriebene Gleichungssystem (4b) vereinfacht sich dann folgendermaßen zum Gleichungssystem (4c), dass nicht mehr jede der *n* Gleichungen *n* Unbekannte enthält. Vielmehr reduziert sich die Zahl der Unbekannten auf *n*=1 für die erste Gleichung, auf *n*=2 Unbekannte für zwei Gleichungen auf *n*=3 Unbekannte bei drei Gleichungen usw.. Durch die Wahl einer Referenzsubstanz kann das Gleichungssystem vereinfacht werden und ist leichter zu lösen, wenn der Extinktionskoeffizient der Referenzsubstanz bekannt ist. Die Konzentration der Referenzsubstanz kann bekannt oder unbekannt sein. Mit dem Gleichungssystem ist es möglich, an einem charakteristischen Meßpunkt, d. h. einer bestimmten Wellenlänge, *n* unbekannte Konzentrationen zu bestimmen, sofern die Verhältnisse der Konzentrationen der *n* urämischen Toxine an einem charakteristischen Meßpunkt, d. h. einer bestimmten Wellenlänge, bekannt sind. Für die Wahl eines oder mehrerer charakteristischer Meßpunkte müssen die im Abschnitt *Auswahlkriterien für einen charakteristischen Meßpunkt* genannten Kriterien zutreffen.

Durch iterative Vorgehensweise kann dieses Gleichungssystem gelöst werden, indem man beispielsweise zuerst bei der Wellenlänge misst, bei der nur Harnsäure im gebrauchten Dialysat absorbiert, und dann zu Wellenlängen wechselt, bei denen im gebrauchten Dialysat (d.h. im Dialysatabfluß) neben Harnsäure im Wesentlichen erst eine weitere Substanz, dann zwei weitere Substanzen bis hin zu *n* weitere Substanzen absorbieren. Somit kann ohne deren direkte Messung die Absorbanz einer Substanz im gebrauchten Dialysat, und mit Kenntnis der jeweiligen Extinktionskoeffizienten, sogar die Konzentration der Substanz im gebrauchten Dialysat bestimmt werden.

Die iterative Vorgehensweise ist jedoch nur eine Möglichkeit, wenn auch eine bevorzugt Möglichkeit, obiges Gleichungssystem zu lösen, welche damit beginnt, dass eine Referenzsubstanz, d.h. ein Referenztoxin bei einer charakteristischen Wellenlänge vermessen wird, wo dieses Toxin die UV-Absorption fast alleine bestimmt oder zumindest zu mindestens 90%, vorzugsweise 94% und insbesondere bevorzugt zu 96% alleine bestimmt. Eine weitere Möglichkeit zur Lösung des Gleichungssystems aus n Gleichungen für n zu bestimmende Konzentrationen von n urämischen Toxinen besteht darin, charakteristische Messpunkte auszuwählen, wo mehrere Toxine das Absorptionsspektrum maßgeblich mitbestimmen. Wählt man beispielsweise zwei Messpunkte, wo dieselben zwei urämischen Toxine die Absorption maßgeblich bestimmen, d.h. zu mindestens 90%, vorzugsweise 94% und insbesondere bevorzugt zu 96% beide zusammen die Gesamtabsorption bestimmen, so sind zwei Varianten denkbar. Falls bei Wellenlänge a das Toxin x die Absorbanz m und bei dieser Wellenlänge das Toxin y die Absorbanz n hat (wobei auch m = n sein kann) und am zweiten Messpunkt bei der Wellenlänge b Toxin x die Absorbanz m/2 hat und das Toxin y die Absorbanz n/2 hat, so sind die Verhältnisse der Absorbanzen der Toxine x und y an beiden Messpunkten, d.h. bei beiden Wellenlängen gleich, so dass sich aus der Messung bei Wellenlänge b keine weitere Information ergibt, d.h. das Gleichungssystem aus 2 Gleichungen mit 2 Unbekannten kann nicht gelöst werden.

Hat hingegen bei Wellenlänge a das Toxin x die Absorbanz m und bei dieser Wellenlänge das Toxin y die Absorbanz n (wobei auch m = n sein kann) und am zweiten Messpunkt bei der Wellenlänge b Toxin x die Absorbanz m/2 und das Toxin y die Absorbanz n/3, so sind die Verhältnisse der Absorbanzen der Toxine x und y an beiden Messpunkten unterschiedlich und das Gleichungssystem aus zwei Gleichungen mit zwei Unbekannten kann gelöst und die Konzentrationen der Toxine x und y können bestimmt werden. Im letzten Fall sind die Messpunkte charakteristisch, d.h. die Spektren beider Toxine ausreichend unterschiedlich, so dass sich bei den weiteren Messpunkten keine redundanten Informationen ergeben.

Somit ist für die Bestimmung der Konzentrationen von n Toxinen wichtig, dass bei n Wellenlängen die Absorption gemessen wird, ohne redundante Informationen zu erhalten. Um somit die Lösung eines Gleichungssystems aus n Unbekannten, d.h. n Konzentrationen von n Toxinen immer auflösen zu können, empfiehlt sich die Messung bei n+z Wellenlängen, wobei z = 1, 2, 3, 4, 5, 6, 7, 8, 9 oder 10 ist.

Für die Bestimmung der jeweiligen Absorbanz wie oben beschrieben, gilt ebenfalls nach Lambert-Beer:

$$A_{\lambda_i} = \log\left(\frac{I_{A,0,\lambda_i}}{I_{A,t,\lambda_i}}\right) = \varepsilon_{\lambda_i} \cdot l \cdot c(t) \quad (5)$$

wobei $A_{\lambda,i}$ die Absorbanz bei einer bestimmten Wellenlänge $\lambda_i$, $I_{RA,0,\lambda i}$ die Intensität am Absorptionsdetektor mit einer Referenzlösung und $I_{A,t,\lambda i}$ die Intensität am Absorptionsdetektor während einer Dialysetherapie zum Zeitpunkt t bei einer bestimmten Wellenlänge $\lambda_i$ darstellt. Wie in Gleichung (1) stellt $\varepsilon_{\lambda,i}$ den wellenlängenabhängigen Extinktionskoeffizienten, $l$ die optische Weglänge und c(t) die jeweilige Konzentration eines Stoffes zum Zeitpunkt t dar.

Durch die Bestimmung der Intensität $I_{A,0,\lambda i}$ mit einer Referenzlösung - idealerweise frisches Dialysat, da hierbei eine evt. Absorbanz nicht durch Toxine hervorgerufen wird - zu Beginn der Dialysetherapie und mit einer kontinuierlichen Bestimmung der Intensität $I_{A,t,\lambda i}$ während der Therapie kann die Absorbanz für eine bestimmte Wellenlänge $\lambda_i$ jederzeit bestimmt werden.

Absorbanzen UV-aktiver urämischer Toxine

Wie bereits oben ausgeführt, ist Harnsäure bei nahezu allen Dialysepatienten im Dialysatabfluß zu erwarten, so dass eine Messung bei $\lambda$=290 nm vorteilhafter Weise standardmäßig durchgeführt werden sollte, um die Harnsäurekonzentration zu bestimmen.

Um die Konzentration von Malondialdehyd zu bestimmen, empfiehlt sich eine Messung bei $\lambda$=266 nm.

Um die Konzentration von Creatinin zu bestimmen, empfiehlt sich eine Messung bei $\lambda$=235 nm.

Somit werden die Absorptionsmessungen bei den folgenden Wellenlängen durchgeführt: $\lambda$=320 nm, $\lambda$=310, $\lambda$=305 nm, $\lambda$=300 nm, $\lambda$=290 nm, $\lambda$=280 nm, $\lambda$=270 nm, $\lambda$=266 nm, $\lambda$=260, $\lambda$=250 nm, $\lambda$=245 nm, $\lambda$=240, $\lambda$=235 nm, $\lambda$=230 nm und $\lambda$=220 nm. Als zu bestimmende urämische Toxine sind Creatinin, Harnsäure, Hippursäure, Indoxylsulfat, 4-Hydroxynonenal, Malondialdehyd, p-Cresol, Phenol, Retinol-bindendes Protein und $ß_2$-Microglobulin-Fragmente und/oder Kombinationen davon bevorzugt.

Bevorzugte Messpunkte sind bei den Wellenlängen $\lambda$=310 nm, $\lambda$=290 nm, $\lambda$=266 nm, $\lambda$=235 nm und $\lambda$=220 nm. Mehr bevorzugte Wellenlängen sind $\lambda$=310 nm, $\lambda$=290 nm, $\lambda$=280 nm, $\lambda$=266 nm, $\lambda$=250 nm, $\lambda$=245 nm, $\lambda$=235 nm und $\lambda$=220 nm. Am meisten bevorzugt sind die Wellenlängen $\lambda$=320 nm, $\lambda$=310, $\lambda$=305 nm, $\lambda$=300 nm, $\lambda$=290 nm, $\lambda$=280 nm, $\lambda$=270 nm, $\lambda$=266 nm, $\lambda$=260, $\lambda$=250 nm, $\lambda$=245 nm, $\lambda$=240, $\lambda$=235 nm, $\lambda$=230 nm, $\lambda$=220 nm und $\lambda$=205 nm.

Pro zu bestimmendes urämisches Toxin ist eine Absorbanzmessung an einem chararkteristischen Messpunkt bevorzugt, d.h. einer bestimmten Wellenlänge, wo dieses urämische Toxin ein Absorptionsmaximum oder zumindest eine hohe Absorption oder eine gute Absorption mit wenig Überlagerung hat. Für die Bestimmung der Konzentration von z.B. 10 Toxinen ergibt sich ein Gleichungssystem mit 10 Variablen, so dass 10 Messungen benötigt werden, wobei die 10 Messungen nicht zu redundanten Informationen führen dürfen und um genaue Messungen zu erhalten an charakteristischen Punkten durchgeführt werden sollten. Für die Wahl eines oder mehrerer charakteristischer Meßpunkte müssen die im Abschnitt *Auswahlkriterien für einen charakteristischen Meßpunkt* genannten Kriterien zutreffen. Zudem sollte nach Möglichkeit in dem gemessenen Bereich nur dieses eine urämische Toxin absorbieren. Ist eine solche Wellenlänge oder ein solcher Wellenlängenbereich für ein bestimmtes urämisches Toxin nicht vorhanden, so wird bei einem Absorptionsmaximum oder einer hohen Absorption dieses urämischen Toxins gemessen, wo eine Überlagerung mit der Absorption eines oder mehrerer anderer urämischer Toxine vorliegt, sofern das eine oder zumindest eines der mehreren anderen urämischen Toxine, welche in dem Messbereich ebenfalls absorbieren, einen anderen Messbereich oder eine andere Wellenlänge mit einem Absorptionsmaximum oder einer hohen Absorption besitzen, welche nicht oder nur unwesentlich durch die Absorption anderer urämischer Toxine überlagert wird. Da insgesamt $n$ Gleichungen mit $n$ Unbekannten zu lösen sind, um die Konzentrationen der $n$ UV-aktiven urämischen Toxine zu bestimmen, wird der Fachmann die Meßpunkte so wählen, dass sich dieses Gleichungssystem lösen lässt. Bei einem zu bestimmenden UV-aktiven Toxin ergibt sich also eine Gleichung mit einer Unbekannten, so dass die Messung bei mindestens einer Wellenlänge im UV-Bereich durchgeführt werden muß. Bei zwei zu bestimmenden UV-aktiven Toxinen ergibt sich eine Gleichung mit zwei Unbekannten, so dass die Messung bei mindestens zwei Wellenlängen im UV-Bereich durchgeführt werden muß, wobei sich keine redundante Information ergeben darf. Bei drei zu bestimmenden UV-aktiven Toxinen ergibt sich eine Gleichung mit drei Unbekannten und einer Messung bei mindestens drei Wellenlängen usw.. Allgemein ergibt sich bei $n$ zu bestimmenden UV-aktiven Substanzen eine Gleichung mit $n$ Unbekannten, so dass die Messung bei mindestens n Wellenlängen im UV-Bereich durchgeführt werden muß und sich keine redundanten Information ergeben dürfen. Für die Auswahl der Wellenlängen ist wichtig, daß die Spektren der urämischen Toxine bekannt sind und sich die Spektren der urämischen Toxine ausreichend unterscheiden. Die Auswahl der Meßpunkte kann beispielsweise anhand lokaler Maxima, lokaler Minima, Flankenpunkten und/oder Wendepunkten der Spektren der urämischen Toxine getroffen werden, sofern diese sich zwischen den zu bestimmenden urämischen Toxinen unterscheiden sollten. Für den Fall, dass sich die lokalen Maxima, lokalen Minima, Flankenpunkten und/oder Wendepunkte der Spektren der urämischen Toxine bei einer bestimmten Wellenlänge überlagern sollten, kann ein charakteristischer Meßpunkt in anderen Bereichen der Spektren der urämischen Toxine wie zum Beispiel in Bereichen der Spektren der urämischen Toxine mit positiver oder negativer Steigung insbesondere zwischen einem Maximum und einem Wendepunkt gewählt werden. Bevorzugt ist, dass an den charakteristischen Meßpunkten des Spektrums des verbrauchten Dialysats mindestens ein zu bestimmendes urämisches Toxin absorbiert. Es ist auch möglich, dass an charakteristischen Meßpunkten, d. h. Wellenlängen, gemessen wird, bei denen sich maximal zwei oder drei oder vier Stoffe überlagern, d. h. dort absorbieren, wobei die Zahl der charakteristischen Meßpunkte, d. h. Wellenlängen, mindestens die Zahl der zu bestimmenden Stoffe beträgt, da sonst das Gleichungssystem nicht gelöst werden kann.

Wird an charakteristischen Meßpunkten, d. h. Wellenlängen, gemessen, bei denen sich maximal zwei oder drei oder vier Stoffe überlagern, d. h. dort absorbieren, kann das Gleichungssystem vereinfacht und leichter gelöst werden. Zusätzlich besteht der Vorteil, dass weniger charakteristische Meßpunkte, d. h. maximal zwei oder drei oder vier, auch weniger Abweichungen bei den Absorbanzen bedingt durch Meßfehler bedeuten. Für den Fall, dass zwei urämische Toxine bestimmt werden sollen, die an zwei charakteristischen Meßpunkten absorbieren, muß sich die Absorbanz der beiden urämischen Toxine an beiden charakteristischen Meßpunkten ausreichend unterscheiden. Die Absorbanz der beiden urämischen Toxine an beiden charakteristischen Meßpunkten ist dann ausreichend unterschiedlich, wenn das Verhältnis der Absorbanzen der zwei urämischen Toxine am ersten der zwei charakteristischen Meßpunkten ungleich dem Verhältnis der Absorbanzen der zwei urämischen Toxine am zweiten der zwei charakteristischen Meßpunkten ist. Ist das Verhältnisse der Absorbanzen der zwei urämischen Toxine am ersten der zwei charakteristischen Meßpunkten gleich dem Verhältnis der Absorbanzen der zwei urämischen Toxine am zweiten der zwei charakteristischen Meßpunkten, kann das Gleichungssystem nicht gelöst werden, weil sich eine redundante Information ergibt. Zur Lösung des Gleichungssystems wird ein weiterer charakteristischer Meßpunkt, d. h. eine weitere Wellenlänge benötigt, wobei das Verhältnis der Absorbanzen der zwei urämischen Toxine am weiteren charakteristischen Meßpunkt ungleich dem Verhältnis der Absorbanzen der zwei urämischen Toxine am zweiten charakteristischen Meßpunkt sein muß. Für $n$ zu bestimmende urämische Toxine müssen folglich mindestens $n$ charakteristische Meßpunkte, d. h. $n$ Wellenlängen, gewählt werden, wobei das Verhältnis der Absorbanzen der n zu bestimmenden urämischen Toxine bei mindestens einem charakteristischen Meßpunkt, d. h, einer der $n$ Wellenlängen, ungleich dem Verhältnis der Absorbanzen der urämischen Toxine bei den anderen charakteristischen Meßpunkten, d. h. anderen Wellenlängen, sein muß. Dies bedeutet, dass an zwei der n Messpunkte das Verhältnis der Absorbanzen der an diesem Messpunkt absorbierenden Toxine nicht identisch oder nahezu identisch sein darf und nach Möglichkeit einen deutlichen Unterschied aufweisen sollte.

Der Extinktionskoeffizient ist für eine bestimmte Substanz bei einer bestimmten Wellenlänge charakteristisch und zum einen von der Temperatur und vom pH-Wert abhängig, zum anderen aber auch vom Lösungsmittel, welches Wechselwirkungen mit den absorbierenden Molekülen eingehen kann. Die Extinktionskoeffizienten der zu bestimmenden UV-aktiven Substanzen sind im Stand der Technik bekannt und können in Standardwerken eingesehen werden, z. B. Lange's Nandbook of Chemistry (14. Ausgabe, Hrsg. J. A. Dean, 1992. McGraw-Hill, Inc., New York), Second Handbook of Chemistry and Physics (56. Ausgabe, Hrsg. R. C. Weast, 1975. CRC Press, Cleveland) oder Third Practical Handbook of Biochemistry and Molecular Biology, Hrsg. D. G. Fasman, 1992. CRC Press, Boston. Die Bestimmung der Extinktionskoeffzienten durch den Fachmann ist ebenso möglich, da die Bestimmung von Extinktionskoeffzienten im Können des Fachmanns liegt.

Messeinrichtung (37) und Strahlungsquelle (1)

Der für die Durchführung der Messungen beschriebene Sensor, der hierin auch als Messeinrichtung oder UV-Sensor (37) bezeichnet wird, ist ähnlich der bereits in der EP 1 083 948 B1 beschriebenen Messeinrichtung. Der Unterschied besteht darin, dass die Lichtquelle (1) nun aus mindestens zwei monochromatischen Lichtquellen besteht oder einer polychromatischen Lichtquelle, welche mit Hilfe eines wellenlängenselektiven Elementes (2) einzeln angesteuert werden kann.

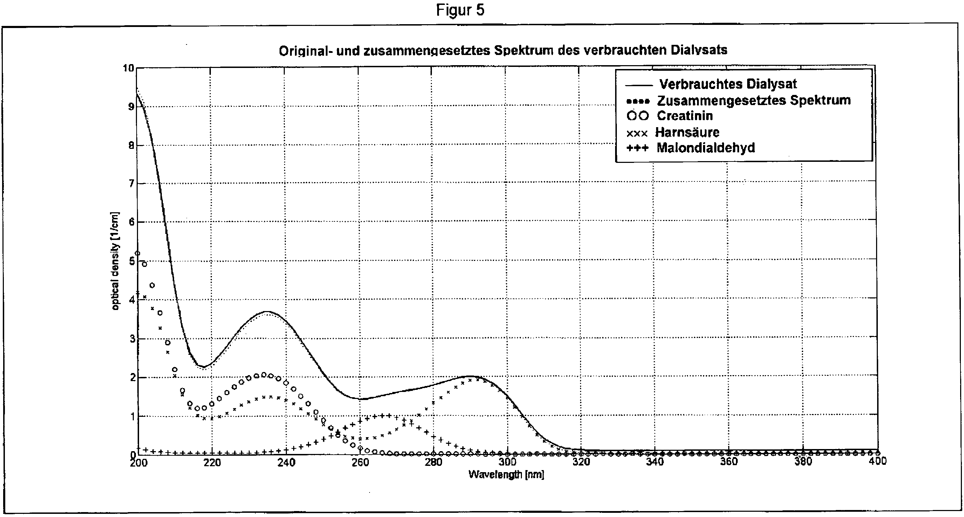

Referenz- (6) und Absorptionsdetektor (5) zeichnen sich vor allem dadurch aus, dass sie das gesamte Spektrum im UV-Bereich zwischen 160 nm und 400 nm Wellenlänge detektieren können. Eine Abbildung eines solchen Sensors ist in Figur 5 zu sehen.

Um die beschriebenen Berechnungen mit einem solchen Sensor während einer Dialysetherapie durchführen zu können, ist ein Verfahren notwendig, welches das Messen der optischen Eigenschaften des verbrauchten Dialysates steuert. Dazu müssen verschiedene gerätetechnische Inhalte umgesetzt werden, welche im Folgenden beschrieben werden.

Die Absorbanzmessungen

Zu Beginn der Dialysetherapie ist eine Kalibrierung des Absorbanz- und Referenzdetektors mit allen - wenigstens aber mit zwei - Wellenlängen nötig. Dazu muss der Sensor (37) mit einer Referenzflüssigkeit gefüllt werden und das Signal am Absorptionsdetektor (5) ermittelt werden. Um ein möglichst starkes Signal am Absorptionsdetektor zu erhalten ist es sinnvoll, die Verstärkerschaltung, welche das Signal am optischen Detektor in ein elektrisches Signal umwandelt, so auszulegen, dass die Intensität am Absorptionsdetektor bei der Kalibrierung mit frischem Dialysat nahe des Maximums der elektrischen Verstärkung liegt, so dass auch das elektrische Signal maximal wird.

Alternativ dazu kann auch die Intensität der LED für jede Wellenlänge variiert werden. Sinnvollerweise sollte die Ansteuerung der LED so gewählt werden, dass der Strom durch die LED unter 50% des maximal zulässigen Stroms bleibt, um die Belastung der LED gering zu halten und so Alterungseffekten vorzubeugen. Dies sollte in Abstimmung mit der Anpassung der elektrischen Verstärkerschaltung für den Absorptionsdetektor während der Kalibrierung erfolgen.

Die zuvor beschriebenen Verfahren in Verbindung mit einer Dialysetherapie sind zum Zeitpunkt der Vorbereitung einer Dialysetherapie durchzuführen. Das Vorbereiten bei einer Dialysetherapie zeichnet sich dadurch aus, dass der extrakorporale Blutkreislauf noch nicht an den Patienten angeschlossen ist, so dass gewährleistet wird, dass sich nach der Dialysataufbereitung, die in der Regel gleich zu Beginn der Vorbereitung geschieht, frisches Dialysat im Abfluss der Dialysemaschine befindet, so dass eine Kalibrierung durchgeführt werden kann.

Ergänzend dazu sollte bei der Kalibrierung der elektrischen Signale, die durch den UV-Sensor im Abfluss der Dialyse erzeugt werden, auch die Temperatur des Dialysates berücksichtigt werden. Da die Temperatur des Dialysates eine kritische Funktion innerhalb der Dialyse darstellt, kann die Temperaturerfassung dazu genutzt werden, um die Richtigkeit der Kalibrierung zu gewährleisten. Dazu ist es notwendig, dass die Kalibrierung erst dann durchgeführt wird, wenn die Temperatur des Dialysates in einem geeigneten Bereich, in der Regel bei ca. 35 bis 37 °C liegt. Die entsprechende Dialysattemperatur wird beim Vorbereiten einer Dialysetherapie durch die Dialysemaschine sichergestellt.

Dies sollte für jede Wellenlänge (jedoch mindestens zwei) des Systems separat durchgeführt werden. Da die Intensität der Lichtquelle und die Sensitivität des Absorptions- bzw. Referenzdetektors von der ausgestrahlten Wellenlänge abhängt, müssen die Signale an beiden Detektoren in der Kalibrierung für die Referenzflüssigkeit bestimmt werden und in einem Speicher hinterlegt werden, auf den für die eigentlichen Messungen im weiteren Verlauf der Dialysetherapie jederzeit zugegriffen werden kann. Während der tatsächlichen Messung innerhalb einer Dialysetherapie zum Zeitpunkt t wird die gemessene Intensität $I_{A,t,\lambda i}$ für eine bestimmte Wellenlänge ermittelt und mittels Gleichung (5) mit dem bereits während der Kalibrierung ermittelten Referenzwert $I_{A,0,\lambda i}$ verknüpft. Dies geschieht einzeln für jede Wellenlänge - jedoch mindestens für zwei Wellenlängen - der Lichtquelle. Hier sei angemerkt, dass es sich bei dem Verfahren nicht um ein Therapieverfahren oder ein Diagnoseverfahren am menschlichen Körper handelt, weil aus den erhaltenen Werten weder eine Therapie noch eine Diagnose abgeleitet werden. Die erfassten Daten dienen lediglich zur Protokollierung des Dialyseverlaufs so wie auch beispielsweise die einwandfreie Funktion einer Pumpe im Dialysegerät überwacht werden kann und bei einer Fehlfunktion oder einem Ausfall entsprechend ersetzt wird, was auch mit einer Therapie oder Diagnose am menschlichen Körper nicht in Verbindung steht. Zudem lassen die erhaltenen Daten Rückschlüsse auf die Qualität der Dialysesitzung zu als auch von neu verwendeten Bauteilen im Dialyseapparat und deren Eignung insgesamt als auch für einen konkreten Patienten.

Die Messung der Absorbanz im Dialysat für unterschiedliche Wellenlängen geschieht nacheinander, wobei die gemessene Intensität $I_{A,t,\lambda i}$ und die Referenzintensität $I_{A,0,\lambda i}$ mittels Gleichung (5) verknüpft werden. Die Referenzintensitäten $I_{A,0,\lambda i}$ sind bereits zuvor in der Kalibrierungsphase ermittelt worden, so dass die Berechnung der jeweiligen Absorbanz $A_{\lambda i}$ für jede Wellenlänge einzeln durchgeführt wird. Des Weiteren können die Ergebnisse der einzelnen Absorbanzmessungen genutzt werden, um mittels des in den Gleichungen (2) bis (5) beschriebenen linearen Gleichungssystems verknüpft zu werden.

Vorzugsweise liegt die Meßdauer für eine Wellenlänge pro Leuchtdiode im Bereich zwischen 1 und 30 Sekunden. Der Wechsel zwischen den Wellenlängen für die Leuchtdiode erfolgt im Millisekunden-/Sekundenbereich und ist von den Herstellerspezifikationen für die jeweilige Leuchtdiode abhängig.

Für im Wesentlichen monochromatische Strahlungsquellen bestehend aus mehreren Lichtquellen wird pro Wellenlänge eine Leuchtdiode benötigt. Bevorzugt liegt die Zahl der Leuchtdioden pro Gehäuse im Bereich von 1 bis 5, bevorzugt im Bereich von 1 bis 8, weiter bevorzugt im Bereich von 1 bis 10 und am meisten bevorzugt im Bereich von 1 bis 16, wobei auch eine höhere Anzahl an Leuchtdioden verwendet werden können, sofern die Gehäusegrößen bzw. die Leuchtdiodengrößen dies zulassen. Auch zwei oder mehr Gehäuse mit einer oder mehreren Leuchtdioden sind möglich.

Für jedes zu bestimmende UV-aktive urämisches Toxin wird bei einer Wellenlänge gemessen. Soll eine Kombination aus verschiedenen Substanzen bestimmt werden, so sind beispielsweise für eine Kombination aus Harnsäure, Creatinin und Malondialdehyd drei Wellenlängen nötig, wie aus dem Gleichungssystem (2) hervorgeht. Dabei ist jede zu bestimmende Substanz eine Unbekannte in der Gleichung (2). Um $n$ Unbekannte zu bestimmen, benötigt man daher $n$ Gleichungen. Daraus ergibt sich wiederum, dass für jede Wellenlänge, bei der die Absorbanz einer Substanz bestimmt werden soll, zu einer neuen Gleichung (2) führt.

Die Absorbanzmessung für den Bereich zwischen 180 und 320 Nanometer erfolgt bei diskreten Wellenlängen. Da sich die Spektren der zu bestimmenden Substanzen hinreichend unterscheiden, werden nur so viele Messpunkte benötigt, wie zu bestimmende Substanzen vorhanden sind. Somit werden die Absorptionsmessungen bei den folgenden Wellenlängen durchgeführt: $\lambda$=320 nm, $\lambda$=305 nm, $\lambda$=290 nm, $\lambda$=280 nm, $\lambda$=266 nm, $\lambda$=245 nm, $\lambda$=235 nm und $\lambda$=220 nm, wenn bis zu acht Substanzen bestimmt werden sollen, und bei $\lambda$=320 nm, $\lambda$=310, $\lambda$=305 nm, $\lambda$=300 nm, $\lambda$=290 nm, $\lambda$=280 nm, $\lambda$=270 nm, $\lambda$=266 nm, $\lambda$=260, $\lambda$=250 nm, $\lambda$=245 nm, $\lambda$=240, $\lambda$=235 nm, $\lambda$=230 nm, $\lambda$=220 nm und $\lambda$=205 nm, wenn bis zu 16 Substanzen und vorzugsweise die 10 erwähnten urämischen Toxine bestimmt werden sollen. Die charakteristischen Meßpunkte werden nach den im Abschnitt *Auswahlkriterien für einen charakteristischen Meßpunkt* genannten Kriterien gewählt. Ebenso können Messungen bei einer Auswahl der vorgenannten Wellenlängen durchgeführt werden. Messungen bei weiteren als den vorgenannten Wellenlängen sind ebenfalls möglich. Für die Auswahl der Wellenlängen ist wichtig, daß die Spektren der urämischen Toxine bekannt sind und sich die Spektren der urämischen Toxine ausreichend unterscheiden, beispielsweise durch unterschiedliche lokale Maxima, lokale Minima oder Wendepunkte oder sich die Spektren der urämischen Toxine an den lokalen Maxima, lokalen Minima oder Wendepunkten unterscheiden. Darüber hinaus müssen die Extinktionskoeffizienten der urämischen Toxine bei dieser Wellenlänge bekannt sein. Die Bestimmung der Extinktionskoeffizienten der urämischen Toxine liegt im Können eines Fachmanns oder können in Standardwerken nachgesehen werden.

Jede Leuchtdiode in der Messeinrichtung wird einzeln angesteuert, d. h. dass Leuchtdioden, bei deren Wellenlänge gerade nicht gemessen wird, ausgeschalten sind. Entsprechend liegt die Dauer eines Messzyklus zwischen 1 bis 30 Sekunden für eine Wellenlänge, bei der gemessen wird. Werden Messungen für zwei Wellenlängen durchgeführt, liegt die Messdauer im Bereich zwischen 2 und 60 Sekunden. Die Dauer der Meßzyklen ist dabei abhängig von der Zahl der Meßpunkte bzw. Wellenlängen.

Bei polychromatischen Strahlungsquellen bestehend aus mehreren Lichtquellen werden alle benötigten Wellenlängen gleichzeitig ausgesandt, wobei mindestens ein Detektor vorhanden ist. Falls nur ein singulärer Detektor vorhanden ist, muß dem Detektor ein abstimmbarer Filter vorgeschaltet sein, wobei der abstimmbare Filter so angesteuert werden muß, dass der abstimmbare Filter alle benötigten Wellenlängen nacheinander passieren lässt. In diesem Falle liegt die Dauer des Messzyklus im Bereich zwischen 10 Sekunden bis fünf Minuten. Die Dauer des Messzyklus ist dabei abhängig davon, wie lange der Filter benötigt, zwischen den Wellenlängen hin und her zu schalten. Sind zwei oder mehr Detektoren mit jeweils unterschiedlichen wellenlängenselektiven Elementen vorhanden, liegt die Dauer eines Messzyklus im Bereich von einer bis dreißig Sekunden. Bevorzugt ist eine Messeinrichtung mit drei Detektoren, wobei jeder Detektor einen Filter aufweist und der Filter des ersten Detektors nur Licht der Wellenlänge $\lambda_1$, der Filter des zweiten Detektors nur Licht der Wellenlänge $\lambda_2$ und der Filter des dritten Detektors nur Licht der Wellenlänge $\lambda_3$ passieren lässt.

Als abstimmbare Filter können Beugungsgitter oder Kristalle verwendet werden, bei denen die Wellenlängenauswahl durch den Einfallswinkel bestimmt wird. Die vorgenannten Filter sind durch einen Schrittmotor, der den Kristall bzw. das Gitter dreht, mikromechanisch abstimmbar. Alternativ können elektronisch abstimmbare Filter verwendet werden, wobei die Stoffeigenschaften durch das Anlegen von Spannung derart manipuliert werden, dass nur Licht mit einer bestimmten optischen Wellenlänge den Kristall oder das Gitter passiert. Bei der Verwendung einer polychromatischen Strahlungsquelle bestehend aus mehreren Lichtquellen mit zwei oder mehr Detektoren, bei denen jeder Detektor einen separaten Filter aufweist, können Standardfilter wie beispielsweise herkömmliche Bandpassfilter für den UV-Bereich verwendet werden. Weitere Ausgestaltungen der Filter liegen im Können des Fachmanns.

Eine Messeinrichtung umfasst oder besteht aus einer Strahlungsquelle und mindestens einem Detektor. Die Strahlungsquelle kann eine oder mehrere polychromatische Lichtquellen sein. Im Falle dass nur eine polychromatische Lichtquelle verwendet wird, sind Strahlungsquelle und Lichtquelle identisch. Die Strahlungsquelle kann aber auch aus zwei oder mehreren polychromatischen Lichtquellen bestehen. Bei polychromatischen Lichtquellen sind entsprechende Filter zu verwenden, um das Spektrum auf die zu messende Wellenlänge zu beschränken. Bevorzugt ist, wenn die Strahlungsquelle aus mehreren monochromatischen Lichtquellen besteht. Monochromatische Lichtquellen benötigen keine Filter. Die monochromatischen Lichtquellen sollten die gewünschten Wellenlängen emittieren, d.h. die charakteristischen Wellenlängen wie beispielsweise $\lambda$=320 nm, $\lambda$=310, $\lambda$=305 nm, $\lambda$=300 nm, $\lambda$=290 nm, $\lambda$=280 nm, $\lambda$=270 nm, $\lambda$=266 nm, $\lambda$=260, $\lambda$=250 nm, $\lambda$=245 nm, $\lambda$=240, $\lambda$=235 nm, $\lambda$=230 nm, $\lambda$=220 nm und $\lambda$=205 nm ($\lambda$ bezeichnet die Wellenlänge).

Wird eine singuläre Strahlungsquelle eingesetzt, muß diese als polychromatische Strahlungsquelle ausgebildet sein. Im Fall einer singulären polychromatischen Strahlungsquelle mit einer singulären Lichtquelle und einem singulärem Detektor müssen die Filter als abstimmbare Filter ausgebildet sein. Die Messungen bei verschiedenen Wellenlängen, insbesondere bei $\lambda$=320 nm, $\lambda$=310, $\lambda$=305 nm, $\lambda$=300 nm, $\lambda$=290 nm, $\lambda$=280 nm, $\lambda$=270 nm, $\lambda$=266 nm, $\lambda$=260, $\lambda$=250 nm, $\lambda$=245 nm, $\lambda$=240, $\lambda$=235 nm, $\lambda$=230 nm, $\lambda$=220 nm und $\lambda$=205 nm erfolgen sequentiell. Bevorzugt ist eine Ausgestaltung der Messeinrichtung mit einer polychromatischen Strahlungsquelle mit einer singulären Lichtquelle und mehreren Detektoren, wobei jeder Detektor mit einem spezifischen Filter versehen ist. In diesem Fall erfolgt die Messung für alle Wellenlängen gleichzeitig. Mehr bevorzugt ist die Ausgestaltung mit einer im Wesentlichen monochromatischen Strahlungsquelle bestehend aus mehreren Lichtquellen mit einem Detektor, wobei zwei oder mehr solcher ausgestalteter Messeinrichtung sequentiell in der Leitung 36 des verbrauchten Dialysats angeordnet sind. Die Messungen bei den verschiedenen Wellenlängen erfolgt im Wesentlichen gleichzeitig. Am meisten bevorzugt ist eine Ausgestaltung der Messeinrichtung mit mindestens zwei im Wesentlichen monochromatischen Strahlungsquellen bestehend aus mehreren Lichtquellen und einem Detektor, wobei die Messungen für die verschiedenen Wellenlängen sequentiell erfolgen. Alternativ können die Messungen für die verschiedenen Wellenlängen gleichzeitig erfolgen, falls mindestens zwei Meßeinrichtungen vorhanden sind, die sequentiell an der das verbrauchte Dialysat führenden Leitung 36 an geordnet sind, und jede der Meßeinrichtung mit mindestens einer im Wesentlichen monochromatischen Strahlungsquelle bestehend aus einer Lichtquelle und einem Detektor ausgestaltet ist.

Bevorzugt ist eine Strahlungsquelle, welche aus 10 monochromatischen Lichtquellen für die Absorptionsmessungen bei den Wellenlängen $\lambda$=320 nm, $\lambda$=305 nm, $\lambda$=290 nm, $\lambda$=280 nm, $\lambda$=266 nm, $\lambda$=245 nm, $\lambda$=240 nm, $\lambda$=235 nm, $\lambda$=230 nm und $\lambda$=220 nm besteht.

Weiter bevorzugt eine Strahlungsquelle, welche aus 12 monochromatischen Lichtquellen für die Absorptionsmessungen bei den Wellenlängen 320 nm, 305 nm, 300 nm, 290 nm, 280 nm, 266 nm, 260 nm, 250 nm, 245 nm, 240 nm, 235 nm und 220 nm oder 320 nm, 305 nm, 300 nm, 290 nm, 280 nm, 266 nm, 250 nm, 245 nm, 240 nm, 235 nm, 230 nm und 220 nm besteht.

Noch weiter bevorzugt eine Strahlungsquelle, welche aus 16 monochromatischen Lichtquellen für die Absorptionsmessungen bei den Wellenlängen λ=320 nm, λ=310, λ=305 nm, λ=300 nm, λ=290 nm, λ=280 nm, λ=270 nm, λ=266 nm, λ=260, λ=250 nm, λ=245 nm, λ=240, λ=235 nm, λ=230 nm, λ=220 nm und λ=205 nm besteht.

Die vorgenannten Strahlungsquellen sind insbesondere geeignet, um die Absorbanz folgender urämischer Toxine zu messen und darus deren Konzentration zu bestimmen: Creatinin, Harnsäure, Hippursäure, Indoxylsulfat, 4-Hydroxynonenal, Malondialdehyd, p-Cresol, Phenol, Retinol-bindendes Protein und ß2-Microglobulin-Fragmente

Wir eine polychromatische Lichtquelle verwendet, dann werden solche Filter verwendet, dass Messungen bei den vorgenannten 10, 12 oder 16 Wellenlängen ermöglicht werden.

Um die Messungen der Intensitäten über einen langen Zeitraum sicherzustellen, muss gewährleistet werden, dass die Lichtquelle die in der Kalibrierung eingestellte Intensität auch während der Dialysetherapie, welche normalerweise ca. vier Stunden dauert, konstant abstrahlt. Neben dem benötigten Strom durch die LED wird auch die Intensität des Referenzdetektors $I_{R,0,\lambda i}$ dazu nach Abschluss der Kalibrierung einer Wellenlänge ebenfalls in einem Speicher hinterlegt. Bei der Durchführung der Messung der Absorbanz einer bestimmten Wellenlänge $\lambda_i$ wird zunächst sicherge-stellt, dass die Intensität des Referenzdetektors $I_{R,t,\lambda i}$ zu einem bestimmten Zeitpunkt t identisch oder nahezu identisch mit dem Kalibrierungswert $I_{R,0,\lambda i}$ ist. Ist dies nicht der Fall, so muss die Intensität der Lichtquelle entsprechend angepasst werden. Hierzu wird eine Regelung der Intensität am Referenzdetektor durchgeführt. Regelgröße ist dabei die aktuell abgestrahlte Intensität einer Wellenlänge der Lichtquelle, die am Referenzdetektor erfasst wird, $I_{R,t,\lambda i}$. Dies stellt gleich-zeitig den IST-Wert der Regelung dar. SOLL-Wert ist die jeweilige Referenzintensität bei der gleichen Wellenlänge aus der Kalibrierung: $I_{R,0,\lambda i}$. Durch den Vergleich von IST- und SOLL-Wert kann nun ein Regelalgorithmus implementiert werden, der die Reglerabweichung $\Delta I = I_{R,0,\lambda i} - I_{R,t,\lambda i}$ minimiert. Dies kann z.B. mit einem PI-Regelglied realisiert werden. Stellglied der Regelung ist somit der Strom durch die Lichtquelle, welche die Intensität der Lichtquelle direkt (in der Regel linear) beeinflusst. Störgrößen des Systems, welche die Reglerabweichung herbeiführen, sind z.B. Temperaturänderungen oder Alterungsprozesse der Lichtquelle. Erst nach der Anpassung des IST-Wertes auf den SOLL-Wert kann mit der Messung der Intensität $I_{A,t,\lambda i}$ am Absorptionsdetektor fortgefahren werden.

Durch die Verwendung von mindestens zwei Wellenlängen ist es weiterhin möglich, eine Plausibilitätsprüfung durchzuführen. Diese Plausibilitätsprüfung dient mehreren Zwecken. Zum einen kann durch die Messung der Absorbanz bei z.B. $\lambda_1$=290 nm und $\lambda_2$=230 nm das Verhältnis $A_{\lambda 2}/A_{\lambda 1}$ gebildet werden. Aus Figur 1 geht hervor, dass die Absorbanz $A_{\lambda 2}$ bei dialysepflichtigen Patienten typischerweise wesentlich größer ist als $A_{\lambda 1}$. Durch die Bildung des Verhältnisses kann somit die Funktionstüchtigkeit des Sensors überprüft werden. Ist z.B. das Verhältnis $A_{\lambda 2}/A_{\lambda 1} < 2$, kann z.B. eine Fehlermeldung an die Dialysemaschine gesendet werden, welche die nicht ordnungsgemäße Funktion des Sensors signalisiert.

Zum anderen kann die Absorbanz unterschiedlicher Wellenlängen $A_{\lambda i}$ dazu genutzt werden, um die Patienten abhängig von Parametern wie z.B. Ernährungszustand o. ä. in unterschiedliche Gruppen einzuteilen. Vasilevsiky und Konoplyov wiesen bereits 2005 in ihrem Artikel "Peculiar character of dialyzate ultraviolet extinction spectra as an indicator of nucleic acid metabolism in humans" (Journal of Biomedical Optics 10(4), 44026, July/August 2005) darauf hin, dass sich Patienten aufgrund des Absorbanzverlaufes des Dialysates in Abhängigkeit der Wellenlänge z. T. stark voneinander unterscheiden. Aus dem beschriebenen Verfahren kann durch die Verwendung von zwei Wellenlängen (beispielsweise $\lambda_1$=290 nm und $\lambda_2$=260 nm) die jeweilige Patientengruppe bestimmt werden. Dabei unterscheidet man Patienten, deren Verhältnis $A_{\lambda 1}/A_{\lambda 2}$ ungefähr gleich 1 ist (vgl. Figur 1), > 1 ist (z.B. größer 1,2) oder deren Verhältnis < 1 ist (z.B. kleiner 0,9). Die Information des Patientenstatus könnte möglicherweise ein Indikator für Parameter wie beispielsweise Ernährungszustand oder Morbidität/Mortalität sein.

Für eine detailliertere Beschreibung der Erfindung sind Zeichnungen mit entsprechenden Referenzen angefügt. Weitere Ziele, Vorteile, Merkmale und Anwendungsmöglichkeiten der vorliegenden Erfindung ergeben sich aus der nachfolgenden Beschreibung der Ausführungsbeispiele anhand der Zeichnungen und Beispiele. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen und deren Rückbeziehung.

**Figurenbeschreibung**

Figur 1: Abhängigkeit der normierten Absorbanz von der eingestrahlten Wellenlänge für ein typisches Dialysatspektrum eines dialysepflichtigen Patienten.

Figur 2: Abhängigkeit der normierten Absorbanz von der eingestrahlten Wellenlänge für den kleinmolekularen Stoff Harnsäure.

Figur 3: Abhängigkeit der normierten Absorbanz von der eingestrahlten Wellenlänge für den kleinmolekularen Stoff Malondialdehyd.

Figur 4: Abhängigkeit der Absorbanz von der eingestrahlten Wellenlänge für den kleinmolekularen Stoff Creatinin. Figur 5: Absorptionsverhalten von Harnsäure, Creatinin und Malondialdehyd in verbrauchtem Dialysat im Vergleich zu den Einzelsubstanzen.

Figur 6: Erfindungsgemäße Vorrichtung.

Figur 7: Beispielhafter Aufbau eines Sensors zur Differenzierung von unterschiedlichen urämischen Toxinen im Dialysat mit einer breitbandigen Lichtquelle, einem Monochromator und einem breitbandigen Detektor.

Figur 8: Beispielhafter Aufbau eines Sensors zur Differenzierung von unterschiedlichen urämischen Toxinen im Dialysat mit einer breitbandigen Lichtquelle, mehreren breitbandigen Detektoren und mehreren schmalbandigen Filtern, welche unterschiedlich ausgelegt sind, wobei auf die Monochromatoren verzichtet werden, falls die Detektoren 1 bis n hinreichend schmalbandig sind.

Figur 9: Beispielhafter Aufbau eines Sensors zur Differenzierung von unterschiedlichen urämischen Toxinen im Dialysat mit einer monochromatischen Lichtquelle und einem Detektor. Zur Realisierung dieser Erfindungsschrift dienen für n Wellenlängen n Sensoren dieser Ausführungsart mit unterschiedlichen, monochromatischen Lichtquellen.

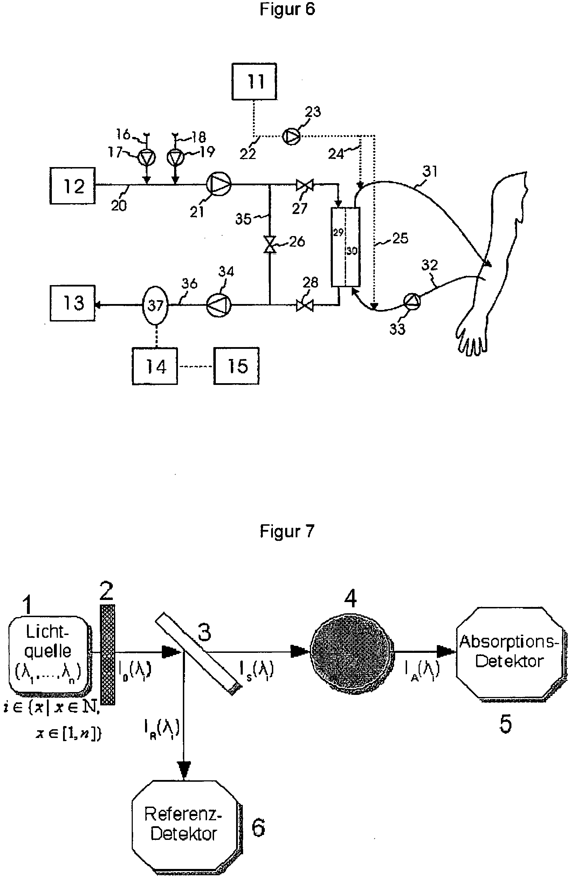

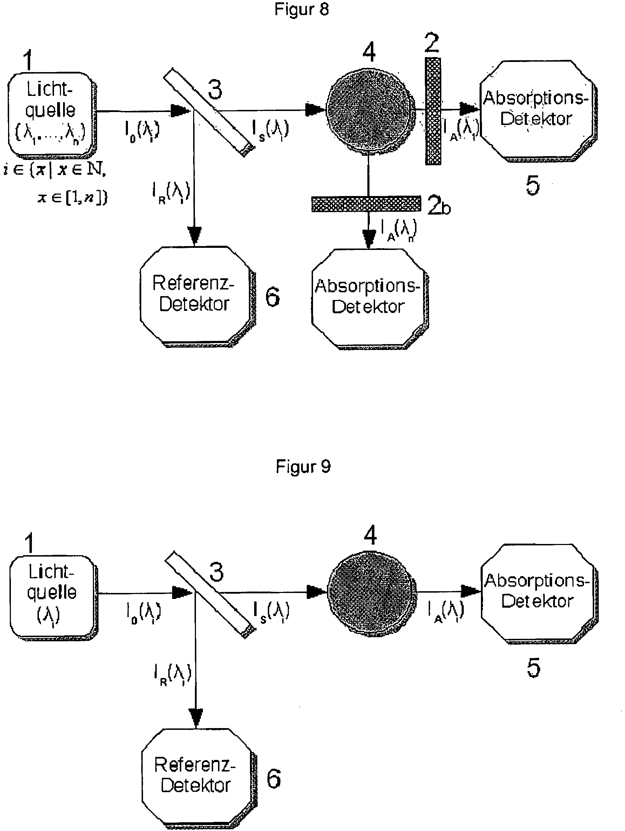

In den Figuren 7 bis 9 bezeichnet 1 stets die Lichtquelle, die je nach Ausführungsform poly- oder monochromatisch ausgebildet ist, 2 stets den Monochromator, 3 stets den Strahlengangteiler, 4 stets die Messstrecke, 5 stets den optischen Detektor, der je nach Ausführungsform schmal- oder breitbandig ausgebildet ist, und 6 stets den Referenzdetektor.

**BEISPIELE**

**Beispiel 1**

Die Dialysatprobe eines dialysepflichtigen Patienten wurde 10 Minuten nach Behandlungsbeginn genommen und deren Absorbanz im Wellenlängenbereich von 200 bis 400 Nanometer spektrophotometrisch bestimmt. Für Wellenlängen größer als $\lambda=340$ nm ist im vorliegenden Fall die Absorbanz zu vernachlässigen. Im Bereich von $\lambda=340$ nm bis $\lambda=290$ nm steigt die Absorbanz zunächst stark an, verhält sich dann im Bereich bis ca. $\lambda=260$ nm stabil, um daraufhin wieder stark anzusteigen. Bei $\lambda=230$ nm ist ein lokales Maximum zu sehen. Bei Wellenlängen kleiner als $\lambda=220$ nm kann ein weiterer Anstieg der Absorbanz beobachtet werden. Dialysatspektren unterschiedlicher Patienten unterscheiden sich in der Regel durch ihre Intensität und den Verlauf der Absorbanz im Bereich zwischen $\lambda=290$ bis $\lambda=255$ nm. Während der Verlauf in dem dargestellten Bild in diesem Bereich nahezu konstant bleibt, kann sich an dieser Stelle auch ein lokales Minimum (mit beispielsweise $A_{\lambda=260\,nm}= 0,5$) oder eine streng monoton steigende Funktion befinden (mit beispielsweise $A_{\lambda=260\,nm}= 1,5$). Figur 1 zeigt das Spektrum der Dialysatprobe aus dem Dialysatabfluß des dialysepflichtigen Patienten.

**Beispiel 2**

Das Absorptionsverhalten von Harnsäure mit einer Konzentration von c=1 mg/l wurde in Wasser für den Wellenlängenbereich von 200 bis 400 Nanometer spektrophotometrisch bestimmt. Figur 2 beschreibt das Absorptionsverhalten von Harnsäure im Bereich von $\lambda=400$ nm bis $\lambda=200$ nm qualitativ. Deutlich sind drei lokale Maxima bei $\lambda=290$ nm, $\lambda=235$ nm und $\lambda=205$ nm zu sehen, wobei die größte Absorption bei den Wellenlängen $\lambda=205$ nm und $\lambda=290$ nm ($A_{\lambda=205\,nm}= 1,5$ bzw. $A_{\lambda=290\,nm}= 0,7$) zu sehen ist.

**Beispiel 3**

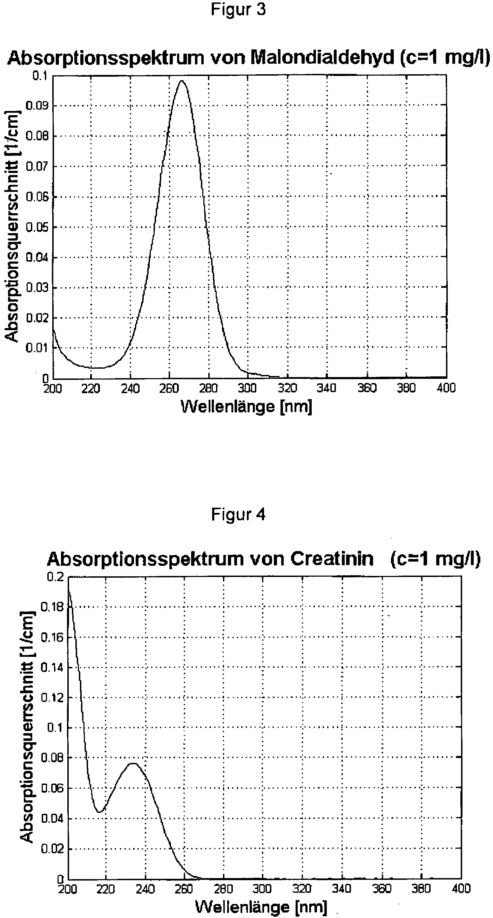

Das Absorptionsverhalten von Malondialdehyd mit einer Konzentration von c=1 mg/l wurde in Wasser für den Wellenlängenbereich von 200 bis 400 Nanometer spektrophotometrisch bestimmt. Figur 3 beschreibt das Absorptionsverhalten von Malondialdehyd im Bereich von $\lambda=400$ nm bis $\lambda=200$ nm qualitativ. Deutlich ist ein lokales Maximum bei $\lambda=266$ nm, ($A\lambda_{=266\,nm}= 1,005$) zu sehen.

**Beispiel 4**

Das Absorptionsverhalten von Creatinin mit einer Konzentration von c=1 mg/l wurde in Wasser für den Wellenlängenbereich von 200 bis 400 Nanometer spektrophotometrisch bestimmt. Figur 4 beschreibt das Absorptionsverhalten von Creatinin im Bereich von $\lambda$=400 nm bis $\lambda$=200 nm qualitativ. Deutlich sind zwei lokale Maxima bei $\lambda$=205 nm, und $\lambda$=235 nm (A=0,19 bzw. A=0,076) zu sehen

**Beispiel 5**

Einem Dialysat wurden 20 mg/l Harnsäure, 30 mg/l Creatinin und 10 mg/l Malondialdehyd hinzugegeben. Das mit Harnsäure, Creatinin und Malondialdehyd substituierte Dialysat wurde anschließend spektral vermessen (kontinuierlich, in 1 nm-Schritten, von 200 bis 400 nm). Gemäß der erfindungsgemäßen Vorrichtung wurde ein Gleichungssystem mit drei Unbekannten bei drei Wellenlängen aufgestellt und daraus die Konzentrationen von Harnsäure, Creatinin und Malondialdehyd berechnet, wobei die Genauigkeit +/- 10% betrug. Figur 5 zeigt das Absorptionsverhalten von Harnsäure, Creatinin und Malondialdehyd in verbrauchtem Dialysat und bestätigt die gute Anwendbarkeit des Verfahrens durch die Nachbildung des verbrauchten Dialysats aus der Linearkombination der genannten Substanzen. Die gezeigten Spektren sind: - verbrauchtes Dialysat, ••• zusammengesetztes Spektrum, ○○○ Creatinin, ××× Harnsäure und +++ Malondialdehyd.

**Beispiel 6**

Die Berechnung der Konzentrationen von Harnsäure, Malondialdehyd und Creatinin wurde für die Proben aus Beispiel 5 durchgeführt. Die charakteristischen Meßpunkte wurden nach den im Abschnitt *Auswahlkriterien für einen charakteristischen Meßpunkt* genannten Kriterien gewählt. Die Absorbanz war bei den Wellenlängen 235, 266 und 290 Nanometer gemessen worden; so ergaben sich folgende Gleichungen:

$$A_{Dialysat\ \lambda=290\,nm} = A_{\lambda=290\,nm\ Harnsäure} + A_{\lambda=290\,nm\ Creatinin} + A_{\lambda=290\,nm\ Malondialdehyd}$$

$$A_{Dialysat\ \lambda=266\,nm} = A_{\lambda=266\,nm\ Harnsäure} + A_{\lambda=266\,nm\ Creatinin} + A_{\lambda=266\,nm\ Malondialdehyd}$$

$$A_{Dialysat\ \lambda=235\,nm} = A_{\lambda=235\,nm\ Harnsäure} + A_{\lambda=235\,nm\ Creatinin} + A_{\lambda=235\,nm\ Malondialdehyd}$$

bzw. mit Gleichung (1) und $l$ = 0,2 *cm* :

$$2,009 = 0,2\ cm \times (\varepsilon_{290\ Harnsäure} \times c_{Harnsäure} + \varepsilon_{290\ Creatinin} \times c_{Creatinin} + \varepsilon_{290\ Malondialdehyd} \times c_{Malondialdehyd})$$

$$1,524 = 0,2\ cm \times (\varepsilon_{266\ Harnsäure} \times c_{Harnsäure} + \varepsilon_{266\ Creatinin} \times c_{Creatinin} + \varepsilon_{266\ Malondialdehyd} \times c_{Malondialdehyd})$$

$$3,685 = 0,2\ cm \times (\varepsilon_{235\ Harnsäure} \times c_{Harnsäure} + \varepsilon_{235\ Creatinin} \times c_{Creatinin} + \varepsilon_{235\ Malondialdehyd} \times c_{Malondialdehyd})$$

Der Extinktionskoeffizient $\varepsilon$ ist substanz- und wellenlängenabhängig, aber für die zu bestimmenden Substanzen bekannt. Die Länge $l$ ist lediglich eine Größe des Messaufbaus. Somit blieben als Unbekannte, und zu bestimmende Größen, die Konzentrationen $c_{Harnsäure}$, $c_{Creatinin}$ und $c_{Malondieldehyd}$, nach denen das Gleichungssystem (4b) aufgelöst wurde. Die Extinktionskoeffizienten $\varepsilon$ waren bekannt bzw. ergaben sich aus Gleichung (1) aus den Substanzspektren (siehe Figuren 1 bis 3) und sind in Tabelle 4 dargestellt:

**Tabelle 4 Extinktionskoeffizienten.**

| Wellenlänge | $\varepsilon_{Harnsäure}$ | $\varepsilon_{Creatinin}$ | $\varepsilon_{Malondialdehyd}$ |
|---|---|---|---|
| 290 nm | 0,4344 l/(cm×mg) | 0,0012 l/(cm×mg) | 0,0510 l/(cm×mg) |
| 266 nm | 0,1114 l/(cm×mg) | 0,0060 l/(cm×mg) | 0,4868 l/(cm×mg) |
| 235 nm | 0,3416 l/(cm×mg) | 0,3809 l/(cm×mg) | 0,0319 l/(cm×mg) |

Löste man das Gleichungssystem, so ergaben sich die Konzentrationen wie folgt:

$$c_{Harnsäure} = 21,8\ \text{mg/l},\ c_{Creatinin} = 27,9\ \text{mg/l und}\ c_{Malondialdehyd} = 10,3\ \text{mg/l}.$$

Die maximale Abweichung lag hier bei circa 9 %.

Das Gleichungssystem konnte, wie bereits erwähnt, vereinfacht werden (siehe Tabelle 5).

**Tabelle 5 Vereinfachte Darstellung der Extinktionskoeffizienten.**

| Wellenlänge | $\varepsilon_{Harnsäure}$ | $\varepsilon_{Creatinin}$ | $\varepsilon_{Malondialdehyd}$ |
|---|---|---|---|
| 290 nm | 0,4344 l/(cm×mg) | **0 l/(cm×mg)** | **0 l/(cm×mg** |
| 266 nm | 0,1114 l/(cm×mg) | **0 l/(cm×mg)** | 0,4868 l/(cm×mg) |
| 235 nm | 0,3416 l/(cm×mg) | 0,3809 l/(cm×mg) | **0 l/(cm×mg)** |

Die Konzentrationen ergaben sich dann zu:

$$c_{Harnsäure} = 21,8\ \text{mg/l},\ c_{Creatinin} = 27,9\ \text{mg/l und}\ c_{Malondialdehyd} = 10,3\ \text{mg/l}.$$

Diese Vereinfachungen reduzierten den Rechenaufwand erheblich unter Erhöhung des maximalen Fehlers auf circa 16%.

Die Ausgangskonzentrationen und die berechneten Konzentrationen sind in Tabelle 6 dargestellt.

**Tabelle 6 Ausgangskonzentrationen und berechnete Konzentrationen.**

| | Harnsäure | Creatinin | Malondialdehyd |
|---|---|---|---|
| Ausgangskonzentration | 20,0 mg/l | 30,0 mg/l | 10,0 mg/l |
| Berechnete Konzentration | 21,8 mg/l | 27,9 mg/l | 10,3 mg/l |
| Abweichung | +9 % | -7% | +3 % |

Die in dieser Rechnung verwendeten Wellenlängen und Substanzen sind beispielhaft. Es versteht sich für einen Fachmann, dass beliebige Kombinationen der hierin aufgeführten urämischen Toxine mit der erfindungsgemäßen Vorrichtung vermessen werden können.

**Beispiel 7**

Nur wenige der weit über 50 verschiedenen urämischen Toxine im verbrauchten Dialysat zeigen tatsächlich optische Aktivität im UV-Bereich. Urämische Toxine mit optischer Aktivität im UV-Bereich umfassen Creatinin, Harnsäure, Hippursäure, Indoxylsulfat, 4-Hydroxynonenal, Malondialdehyd, p-Cresol, Phenol, Retinol-bindendes Protein und ß$_2$-Microglobulin-Fragmente und/oder Kombinationen davon. Diese urämischen Toxine unterscheiden sich in ihren optischen Eigenschaften, vor allem in ihrer Wellenlänge, zumeist stark. Prinzipiell ist es daher möglich, aus eine Mischung von mehreren urämischen Toxinen die jeweilige Konzentration zu bestimmen, solange die Spektren hinreichend unterschiedlich sind und an mindestens so vielen Wellenlänge die Absorption bestimmt wird, wie zu bestimmende UV-aktive Substanzen im verbrauchten Dialysat vorkommen. Um eine zusätzliche Sicherheit in das System zu bringen und die Genauigkeit des Verfahrens zu erhöhen, kann auch bei mehr Wellenlängen gemessen werden, als Substanzen vorhanden sind, welche die Absorptionsmessung dominieren. Ein solches System heißt überbestimmt. Prinzipiell ist es sinnvoll, an Stellen im Spektrum zu messen, an welchen das Absorptionssignal besondere Merkmale aufweist. So z.B. sind die charakteristischen Wellenlängen im verbrauchten Dialysat 320 nm, 305 nm, 290 nm, 280 nm, 266 nm, 245 nm, 235 nm und 220 nm. Jedoch sind weitere Wellenlängen prinzipiell denkbar. Mit der vorgenannten Wellenlängenkombination kann die Konzentration von bis zu acht optisch aktiven, urämischen Toxinen aus dem verbrauchten Dialysat bestimmt werden. Sollte bei den Wellenlängen $\lambda=320$ nm, $\lambda=310$, $\lambda=305$ nm, $\lambda=300$ nm, $\lambda=290$ nm, $\lambda=280$ nm, $\lambda=270$ nm, $\lambda=266$ nm, $\lambda=260$, $\lambda=250$ nm, $\lambda=245$ nm, $\lambda=240$, $\lambda=235$ nm, $\lambda=230$ nm, $\lambda=220$ nm und $\lambda=205$ nm gemessen werden, können bis zu 16 Substanzen bestimmt werden. Die charakteristischen Meßpunkte müssen die im Abschnitt *Auswahlkriterien für einen charakterisfischen Meßpunkt* genannten Kriterien erfüllen.

Diese Menge an urämischen Toxinen, welche sich im Spektrum z. T. stark voneinander unterscheiden und das Absorptionsspektrum signifikant beeinflussen, ist in der Dialyse realistisch. Daneben gibt es natürlich noch eine Vielzahl weiterer urämischer Toxine, welche jedoch keinen Einfluss auf die Bestrahlung mit UV-Licht aufweisen, d. h. nicht UV-aktiv sind und daher die UV-Messungen auch nicht stören. In der Dialyse wurde Blut, welches mit urämischen Toxinen angereichert ist, im Dialysator des extrakorporalen Schlauchsystems gereinigt. Die Giftstoffe traten in eine Waschlösung über, in der sie bedingt durch die unterschiedlichen Blut- und Dialysatflussraten zunächst verdünnt und abschließend ausgespült wurden. Beim Entsorgen passierten sie ein Schlauchsegment, welches mit ultraviolettem Licht bestrahlt wurde. Je nach Ausführungsform wird dort die Absorption der wässrigen Flüssigkeit für mehrere Wellenlängen , z. B. 320 nm, 305 nm, 300 nm, 290 nm, 280 nm, 266 nm, 250 nm, 245 nm, 240 nm, 235 nm und 220 nm, vermessen. Die charakteristischen Meßpunkte müssen die im Abschnitt *Auswahlkriterien für einen charakteristischen Meßpunkt* genannten Kriterien erfüllen. Zusammen mit den im Speicher der Auswerteeinheit hinterlegten Stoffkonstanten, welche wellenlängenspezifisch sind (vgl. Tabelle 1 und Beispiel 1) kann so ein Gleichungssystem aus $n$ Gleichungen und bis zu $n$ Unbekannten aufgestellt und gelöst werden, wobei $n$ die minimale Anzahl der Messpunkte und der zu bestimmenden Konzentrationen ist (vgl. Beispiel 1). Im vorliegenden Fall wurde zur Bestimmung von 9 urämischen Toxinen bei 11 Wellenlängen gemessen, um selbst beim Auftreten von zwei redundanten Informationen das Gleichungssystem auflösen zu können.

Eine Validierung des Systems geschah durch eine gleichzeitige HPLC-Analyse. Üblicherweise ist die Konzentration urämischer Toxine im verbrauchten Dialysat um den Faktor 10 geringer als im Blut. Dies hängt zum einen mit der Verdünnung, bedingt durch Blut- und Dialysatfluss zusammen, wird zum anderen aber auch von der Reinigungsleistung des Dialysators beeinflusst, welche je nach Stoffgröße üblicherweise < 90% ist, je nach Blutfluss und Dialysator. Physiologische Parameter wie Rezirkulation im Shunt des Dialysepatienten reduzieren die Konzentration urämischer Toxine im Dialysat weiter. Bedingt durch die starke Verdünnung wurden daher nur die urämischen Toxine von der Messeinrichtung erfasst, welche schon im unverdünnten Zustand eine messbare optische Aktivität aufweisen. Die im Stand der Technik bekannten Konzentrationen der UV-aktiven urämischen Toxine im Blutplasma bzw. Blutserum sind in Tabelle 7 gezeigt.

**Tabelle 7 Konzentrationen der UV-aktiven urämischen Toxine.** Modifiziert nach Vanholder et al. (2003).

| UV-aktives urämisches Toxin | Normale Konzentration ($c_N$) | Mittlere bzw. mediane urämische Konzentration ($c_U$) | Maximale Konzentration ($c_{MAX}$) |
|---|---|---|---|
| Creatinin (mg/l) | <12,0 | 136,0±46,0 | 240,0 |
| Harnsäure (mg/l) | <67,2 | 83,4±44,5 | 146,7 |
| Hippursäure (mg/l) | <5,0 | 247,0±112,0 | 471,0 |
| Indoxylsulfat (mg/l) | 0,6±5,4 | 53,0±91,5 | 236,0 |
| Malondialdehyd ($\mu$g/l) | 257,7±81,7 | 428,8±170,4 | 769,6 |
| *p*-Cresol (mg/l) | 0,6±1,0 | 20,1±10,3 | 40,7 |
| Phenol (mg/l) | 0,6±0,2 | 2,7±3,9 | 10,5 |
| Retinof-bindendes Protein (mg/l) | <80 | 192,0±78,0 | 369,2 |
| ß$_2$-Microglobulin-Fragmente (mg(l) | <2,0 | 55,0±7,9 | 100,0 |

Setzt man ein Gleichungssystem analog zu Beispiel 6 an, bei welchem die Absorption bei mindestens 9 Wellenlängen bestimmt wird (z.B. λ=320 nm, λ=305 nm, λ=290 nm, λ=280 nm, λ=266 nm, λ=250 nm, λ=240, λ=230 nm und λ=220 nm), so lässt sich bei der Vermeidung redundanten Informationen das Gleichungssystem lösen und die Konzentrationen der 9 urämischen Toxine bestimmen. Im vorliegenden Fall wurde die Absorption bei den 11 Wellenlängen 320 nm, 305 nm, 300 nm, 290 nm, 280 nm, 266 nm, 250 nm, 245 nm, 240 nm, 235 nm und 220 nm bestimmt. Nach Anwendung des Gleichungssystems nach Gleichung (1) (vgl. hierzu auch Gleichungssystem in Beispiel 6) ergaben sich folgende Werte:

| Stoff | Ausgangskonzentration | berechnete Konzentration | Abweichung |
|---|---|---|---|
| Creatinine | 80,3 mg/l | 75,7 mg/l | 5,7 % |
| Harnsäure | 25,4 mg/l | 21,2 mg/l | 16,5 % |
| Hippursäure | 90,9 mg/l | 83,7 mg/l | 7,9 % |
| Indoxylsulfate | 15,4 mg/l | 12,5 mg/l | 18,8 % |
| Malondialdehyd | 255,3 μg/l | 250,0 μg/l | 2,1 % |
| p-Cresol | 5,1 mg/l | 5,8 mg/l | 13,7 % |
| Phenol | 0,6 mg/l | 0,8 mg/l | 33,3 % |
| Retinol-binding Protein | 25,7 mg/l | 28,7 mg/l | 11,6 % |
| $\beta_2$-Microglobulin | 10,5 mg/l | 8,7 mg/l | 17,1 % |

Diese Messungen wurden im verbrauchten Dialysat durchgeführt und liegen deshalb z.T. deutlich unter den Konzentrationen der Blutkonzentrationen.

**Beispiel 8**

Einen Aufbau der Dialysevorrichtung, mit der das Verfahren umgesetzt wird, zeigt Figur 6. Auf der Dialysatseite ist dabei mindestens eine optische Messeinrichtung 37, die hierin auch als UV-Sensor bezeichnet wird, vorhanden. Das Blut eines Patienten wird vom Patienten in einen extrakorporalen Kreislauf geführt. Das Blut fließt durch einen Schlauch 32 in die blutseitige Kammer 30 eines Dialysators und wird durch einen Schlauch 31 zum Patienten zurückgeführt. Die Flußrate des Blutkreislaufs wird durch eine Blutpumpe 33 kontrolliert. Die Dialyselösung besteht aus einer Reihe physiologisch relevanter Stoffe, die in Wasser gelöst vorliegen, so daß diese wegen des fehlenden Konzentrationsgradienten dem Blut während des Dialysevorgangs nicht entzogen werden. Deshalb umfasst die in Figur 5 offenbarte Dialysevorrichtung eine Wasserzuführung 12, zwei Zuführungen 16 und 18 für Konzentrate physiologisch relevanter Stoffe, die in Wasser gelöst vorliegen, und zwei Pumpen 17 und 19. Der Wasserfluss bestimmt zusammen mit den Konzentratfluss oder den Konzentratflüssen die Zusammensetzung der Dialyselösung. Über den Dialysatkreislauf 20 wird die Dialyselösung der Dialysekammer 29 des Dialysators, die von der Blutkammer 30 durch eine semipermeable Membran getrennt ist, zugeführt. Die Dialyselösung wird dabei von einer Pumpe 21 dem Dialysator zugeführt. Eine weitere Pumpe 34 saugt die Dialysierflüssigkeit und das aus dem Blut entfernte Ultrafiltrat an. Eine Umgehungsverbindung 35 ist zwischen den Pumpen 21 und 34 angeordnet. Ebenfalls sind mehrere Ventile 26, 27 und 28 vorhanden, um den Dialysatfluß zu kontrollieren. Eine Leitung 36 führt das verbrauchte Dialysat zu einem UV-Sensor 37 mit einer Strahlungsquelle 1 bestehend aus vier Lichtquellen für im Wesentlichen monochromatische elektromagnetische Strahlung der Wellenlängen λ=290 nm, λ=266 nm, λ=235 nm und λ=205 nm, die einzeln angesteuert werden, der die Absorbanz des verbrauchten Dialysates misst, wobei der UV-Sensor 37 über eine Schnittstelle mit einem Computer 14 verbunden ist. Die charakteristischen Meßpunkte müssen die im Abschnitt *Auswahlkriferien für einen charakteristischen Meßpunkt* genannten Kriterien erfüllen. Der Computer 14 verarbeitet die Messdaten. Das Ergebnis der Datenverarbeitung wird auf einer Vorrichtung 15 angezeigt und/oder gedruckt, wobei die Vorrichtung 15 mit dem Computer 14 über eine Schnittstelle verbunden ist. Die Leitung 36 führt nach der Messung mit dem UV-Sensor 37 das verbrauchte Dialysat zum Ablaufsystem 13. Die gepunkteten Linien 22, 24 und 25 stellen eine Adaptierung der offenbarten Vorrichtung für Behandlungen mittels Hämodiafiltration dar. Die Ersatzflüssigkeit wird von einer Ersatzflüssigkeitsquelle 11 bereitgestellt, fließt durch den Schlauch 22 und wird durch die Pumpe 23 in den blutzuführenden Schlauch des Patienten gepumpt. Im Falle einer Postdilutions-Hämodiafiltration führt die Leitung 24 die Ersatzflüssigkeit zur venösen Leitung des extrakorporalen Blutsystems. Bei einer Prädilutions-Hämodiafiltration werden sowohl die Leitung 24 als auch die Leitung 25 verwendet. Der Computer 14 kontrolliert alle in Figur 5 gezeigten Elemente mittels geeigneter Schnittstellen, wobei die besagten Schnittstellen wegen mangelnder Übersichtlichkeit nicht dargestellt sind. Der Computer 14 sammelt Informationen über andere Parameter der Dialysevorrichtung, z. B. Blutfluß, Dialysatfluß und/oder Behandlungszeit. Diese Parameter werden zusammen mit den gemessenen Daten verarbeitet.

Die in diesem Beispiel offenbarte Dialysevorrichtung wird mit weiteren bestimmungsgemäßen Mitteln bereitgestellt, wie sie für Dialysevorrichtungen gebräuchlich sind. Diese weiteren Mittel sind nicht offenbart, da sie zur Durchführung des offenbarten erfindungsgemäßen Verfahrens nicht relevant sind.

Die erhaltenen Absorbanzkurven werden in einer Patientenkarte gespeichert, die mit dem Computer 14 verbunden ist, oder in einer Datenbank, die im Computer 14 implementiert ist. Die Zahl der zu speichernden und gespeicherten Absorbanzkurven ist variabel und hängt der Speicherkapazität des Mediums ab. Bei einer bevorzugten Ausführungsform werden die letzten 20 Behandlungen in einer geeigneten Speicherkarte archiviert. Die gespeicherten Behandlungsdaten werden durch ein *First In-First Out*-Verfahren (FIFO) überschrieben. Zusätzlich kann eine durch den Anwender oder behandelnden Arzt zu bestimmende Behandlung als nichtüberschreibbar definiert werden. In diesem Fall bleiben die Behandlungsdaten erhalten, bis die Behandlungsdaten wieder als überschreibbar definiert werden. Die Speicherung der Dialyseleistung (Kt/V) oder Kurven für die Harnstoffreduktionsrate (*urea reduction rate*, URR) ist ebenfalls möglich.

**Beispiel 9**

Eine Ausgestaltung des Sensoraufbaus 37 zum Messen der Absorption bei mehreren Wellenlängen zeigt Figur 7. (1) stellt dabei eine Lichtquelle dar, die entweder aus mehreren (mindestens zwei) monochromatischen Wellenlängen besteht, die einzeln angesteuert werden, oder als eine breitbandige Lichtquelle ausgelegt ist. Im letzten Fall ist ein wellenlängenselektives Element (2) nötig, das gesteuert werden kann, um einzelne Wellenlängen herauszufiltern. (3) stellt einen Strahlengangteiler (3) dar, der den Strahlengang $I_0(\lambda_i)$ in zwei Teile aufteilt: $I_R(\lambda_i)$ und $I_S(\lambda_i)$. Der Anteil $I_R(\lambda_i)$ trifft direkt auf den Referenzdetektor (6), während der Anteil $I_S(\lambda_i)$ auf die zu untersuchende Probe (4) trifft. Abhängig von der Absorption der Probenflüssigkeit trifft eine Intensität $I_A(\lambda_i)$ auf den Absorptionsdetektor (5), welche kleiner oder gleich der ursprünglichen Intensität $I_S(\lambda_i)$ ist. Die Anzahl der Wellenlängen *n*, die in dem Sensor eingesetzt werden könnten, ist prinzipiell beliebig, sollte aber mindesten zwei betragen (mindestens i=[1,2]).

**Beispiel 10**

Ein dialysepflichtiger Patient wurde mit einer Vorrichtung gemäß Beispiel 7 behandelt, wobei der Sensoraufbau zum Messen der Absorption gemäß Beispiel 8 ausgestaltet war. Eine Dialysatprobe des dialysepflichtigen Patienten wurde 10 Minuten nach Behandlungsbeginn genommen und deren Absorbanz spektrophotometrisch bei den Wellenlängen $\lambda$=290 nm, $\lambda$=266 nm und $\lambda$=235 nm bestimmt. Anschließend wurden die Konzentrationen an Harnsäure, Creatinin und Malondialdehyd gemäß Beispiel 6 berechnet. Die berechneten Konzentrationen waren:

$$c_{Harnsäure} = 40{,}0 \text{ mg/l}, \quad c_{Creatinin} = 65{,}3 \text{ mg/l und } c_{Malondialdehyd} = 250 \text{ μg/l}.$$

Die charakteristischen Meßpunkte wurden nach den in Abschnitt *Auswahlkriterien für einen charakteristischen Meßpunkt* genannten Kriterien ausgewählt.

**Patentansprüche**

1. Vorrichtung zur extrakorporalen Blutbehandlung mit

    - einem Dialysator, der durch eine semipermeable Membran in eine erste und zweite Kammer (29, 30) geteilt ist, wobei die erste Kammer (29) in einem Dialysierflüssigkeitsweg angeordnet ist und die zweite Kammer (30) mittels einer Blutzuführleitung (32) und einer Blutabführleitung (31) mit dem Blutkreislauf eines Patienten verbindbar ist,
    - einem Zulauf (20) für frische Dialysierflüssigkeit,
    - einem Ablauf (36) für verbrauchte Dialysierflüssigkeit,
    - einer in dem Ablauf (36) angeordneten Messeinrichtung (37), wobei die Messeinrichtung (37) eine Strahlungsquelle (1) für elektromagnetische UV-Strahlung aufweist,
    - wobei die Strahlungsquelle (1) entweder aus mindestens zwei monochromatischen Lichtquellen oder mindestens einer polychromatischen Lichtquelle mit Monochromatoren zur Erzeugung monochromatischer UV-Strahlung besteht,
    - einer Mikroprozessoreinheit (14), einer Speichereinheit sowie einer Ausgabeeinheit (15), **dadurch gekenn-**

zeichnet, dass

die Messeinrichtung (37) dazu ausgebildet ist, im Wesentlichen monochromatische elektromagnetische UV-Strahlung unterschiedlicher Wellenlängen zu erzeugen und durch den Ablauf (36) für verbrauchte Dialysierflüssigkeit zu führen, wobei wenigstens ein Detektorsystem (5) zur Detektion der Intensität oder Absorbanz der durch den Ablauf (36) für verbrauchte Dialysierflüssigkeit hindurch tretenden im Wesentlichen monochromatischen elektromagnetischen UV-Strahlung vorgesehen ist und in der Speichereinheit ein Gleichungssystem $A_{\lambda i} = \sum_{j=1}^{n} A_j$ hinterlegt ist, wobei $A_{\lambda,i}$ die gesamte Absorbanz eines Stoffgemisches bei einer vorgegebenen Wellenlänge $\lambda_i$, $A_j$ die Absorbanz eines einzelnen Stoffes innerhalb des Stoffgemisches und n die Anzahl der interessierenden Komponenten innerhalb eines Stoffgemisches, welche zur Absorbanz beitragen, bezeichnen.

2. Vorrichtung nach Anspruch 1, **dadurch gekennzeichnet, dass** die Strahlungsquelle (1) bestehend aus mehreren Lichtquellen zur Emission elektromagnetischer Strahlung im Bereich von 180 nm bis 380 nm ausgebildet ist.

3. Vorrichtung nach Anspruch 1 oder 2, **dadurch gekennzeichnet, dass** die Strahlungsquelle (1) eine Mehrzahl von Leuchtdioden aufweist, welche jeweils zur Erzeugung einer im Wesentlichen monochromatischen elektromagnetischen UV-Strahlung ausgebildet sind.

4. Vorrichtung nach Anspruch 1 oder 2, **dadurch gekennzeichnet, dass** die Strahlungsquelle (1) zur Erzeugung polychromatischer elektromagnetischer Strahlung ausgebildet ist und zur Erzeugung der im Wesentlichen monochromatischen elektromagnetischen Strahlung entsprechende Monochromatoren, insbesondere nur für eine spezifische Wellenlänge durchlässige optische Filter oder ein Bandpassfilter mit mehreren Passbereichen vorgesehen sind.

5. Vorrichtung nach einem der vorhergehenden Ansprüche, **dadurch gekennzeichnet, dass** die Mikroprozessoreinheit (14) dazu ausgebildet ist, das Gleichungssystem $A_{\lambda i} = \sum_{j=1}^{n} A_j$ unter Anwendung der Gleichungen $A_{\lambda_i} = \varepsilon_{j,\lambda_i} \cdot l \cdot c_j$ (1) und n Messpunkten und der Absorbanz $A_1$ einer Referenzsubstanz bei einer Referenzwellenlänge $\lambda_1$ zu lösen, wobei $A_j$ für die Absorbanz der Substanz $j$ bei einer bestimmten Wellenlänge $\lambda_i$, $\varepsilon_{\lambda_i}$ den wellenlängenabhän-gigen Extinktionskoeffizienten, $l$ die optische Weglänge und $c_j$ die jeweilige Konzentration der Substanz beschreibt.

6. Vorrichtung nach einem der Ansprüche 1 bis 4, **dadurch gekennzeichnet, dass** durch den Mikroprozessor (14) mit Hilfe einer Referenzabsorbanzkurve einer Referenzsubstanz und folgendem, in der Speichereinheit abgelegtem Gleichungssystems, die Bestimmung von Absorbanzen und/oder Konzentrationen urämischer Toxine im Ablauf (36) vereinfacht wird

$$A_{\lambda i}(Referenzsubstanz) \approx \frac{A_{\lambda 1}(Referenzsubstanz)}{konst_i} + \sum_{j=2}^{n} \varepsilon_{j,\lambda_i} \cdot l \cdot c_j = \frac{A_{\lambda 1}(Referenzsubstanz)}{konst_i} + \sum_{j=2}^{n} A_j \quad ,$$

wobei die Referenzsubstanz bei der Wellenlänge $\lambda_i$ in verbrauchter Dialysierflüssigkeit annähernd ausschließlich absorbiert, wobei $konst_i$ das Verhältnis der Absorbanz der reinen Referenzsubstanz bei der Referenzwellenlänge $\lambda_1$ zu der Absorbanz der reinen Referenzsubstanz bei der Wellenlänge $\lambda_i$ ist.

7. Vorrichtung nach Anspruch 5 oder 6, **dadurch gekennzeichnet, dass** die Referenzsubstanz ausgewählt wird aus der Gruppe umfassend die UV-aktiven urämischen Toxine Creatinin, Harnsäure, Hippursäure, Indoxylsulfat, 4-Hydroxynonenal, Malondialdehyd, p-Cresol, Phenol, Retinol-bindendes Protein und ß2-Microglobulin-Fragmente und/oder Kombinationen davon.

8. Vorrichtung nach Anspruch 7, **dadurch gekennzeichnet, dass** als Referenzsubstanz Harnsäure verwendet wird.

9. Vorrichtung nach einem der Ansprüche 5 bis 8, **dadurch gekennzeichnet, dass** die Referenzwellenlänge $\lambda_1$ im

Bereich zwischen 280 nm und 300 nm liegt.

**10.** Vorrichtung nach einem der vorhergehenden Ansprüche, **dadurch gekennzeichnet, dass** als Strahlungsquelle (1) 10 monochromatische Lichtquellen verwendet werden.

**11.** Vorrichtung nach einem der vorhergehenden Ansprüche, **dadurch gekennzeichnet, dass** die Detektion der Intensität oder Absorbanz bei den Wellenlängen $\lambda=320$ nm, $\lambda=310$, $\lambda=305$ nm, $\lambda=300$ nm, $\lambda=290$ nm, $\lambda=280$ nm, $\lambda=270$ nm, $\lambda=266$ nm, $\lambda=260$, $\lambda=250$ nm, $\lambda=245$ nm, $\lambda=240$, $\lambda=235$ nm, $\lambda=230$ nm, $\lambda=220$ nm und $\lambda=205$ nm erfolgt.

**Claims**

**1.** Apparatus for the extracorporeal blood treatment having

 - a dialyzer which is separated by a semipermeable membrane into a first and second chamber (29, 30), wherein the first chamber (29) is arranged in a dialysate pathway and the second chamber (30) is connectable to the blood circulation of a patient by means of a blood supply conduit (32) and a blood return conduit (31),
 - an inlet (20) for fresh dialysate,
 - an outlet (36) for spent dialysate,
 - a measuring device (37) arranged in the outlet (36), wherein the measuring device (37) has a radiation source (1) for electromagnetic UV radiation,
 - wherein the radiation source (1) consists of either at least two monochromatic light sources or at least one polychromatic light source with monochromators for the generation of monochromatic UV radiation,
 - a microprocessor unit (14), a storage unit as well as an output unit (15), **characterized in that**

the measuring device (37) is designed to generate substantially monochromatic electromagnetic UV radiation of different wavelengths and to direct it through the outlet (36) for spent dialysate, wherein at least one detector system (5) is provided for the detection of the intensity or the absorption of the substantially monochromatic electromagnetic UV radiation passing through the outlet (36) for spent dialysate and a system of equations $A_{\lambda i} = \sum\limits_{j=1}^{n} A_j$ is deposited in the storage unit, wherein $A_{\lambda i}$ is the total absorption of a mixture of compounds at a predetermined wavelength $\lambda_i$, $A_j$ is the absorption of a single compound within the mixture of compounds and $n$ is the number of the interesting components within the mixture of compounds, which contribute to the absorption.

**2.** Apparatus according to claim 1, **characterized in that** the radiation source (1) consisting of several light sources is designed for the emission of electromagnetic radiation in the range of 180 nm to 380 nm.

**3.** Apparatus according to claim 1 or 2, **characterized in that** the radiation source (1) has a plurality of light emitting diodes, which are designed each for the generation of a substantially monochromatic electromagnetic UV radiation.

**4.** Apparatus according to claim 1 or 2, **characterized in that** the radiation source (1) is designed for the generation of polychromatic electromagnetic radiation and adequate monochromators, in particular optical filters pervious for only a specific wavelength or a band-pass filter with several pass ranges, are provided for the generation of the substantially monochromatic electromagnetic radiation.

**5.** Apparatus according to one of the preceding claims, **characterized in that** the microprocessor unit (14) is designed to solve the system of equations $A_{\lambda i} = \sum\limits_{j=1}^{n} A_j$ using the equations $A_{\lambda i} = \varepsilon_{j,\lambda_i} \cdot l \cdot c_j$ (1) and $n$ measuring points and

the absorption $A_1$ of a reference substance at a reference wavelength $\lambda_1$, wherein $A_j$ describes the absorption of the substance $j$ at a certain wavelength $\lambda_i$, $\varepsilon_{\lambda_i}$, describes the wavelength-dependent extinction coefficient, $l$ describes the optical path length and $c_j$ describes the respective concentration of the substance.

**6.** Apparatus according to one of the claims 1 to 4, **characterized in that** by means of the microprocessor (14) with the aid of a reference absorption curve of a reference substance and the following system of equations stored in the storage unit, the determination of absorptions and/or concentrations of uremic toxins in the outlet (36) is simplified

$$A_{\lambda i}(dialysate) \approx \frac{A_{\lambda 1}(\text{reference substance})}{const_i} + \sum_{j=2}^{n} \varepsilon_{j,\lambda_i} \cdot l \cdot c_j = \frac{A_{\lambda 1}(\text{reference substance})}{const_i} + \sum_{j=2}^{n} A_j$$

wherein the reference substance absorbs at the wavelength $\lambda_j$ in spent dialysate almost exclusively, wherein $const_i$ is the ratio of the absorption of the pure reference substance at the reference wavelength $\lambda_1$ to the absorption of the pure reference substance at the wavelength $\lambda_j$.

7. Apparatus according to claim 5 or 6, **characterized in that** the reference substance is selected from the group comprising the UV active uremic toxins creatinine, uric acid, hippuric acid, indoxyl sulfate, 4-hydroxynonenal, malondialdehyde, p-cresol, phenol, retinol-binding protein and ß2-microglobulin-fragments and/or combinations thereof.

8. Apparatus according to claim 7, **characterized in that** uric acid is used as reference substance.

9. Apparatus according to one of the claims 5 to 8, **characterized in that** the reference wavelength $\lambda_1$ is in the range between 280 nm and 300 nm.

10. Apparatus according to one of the preceding claims, **characterized in that** 10 monochromatic light sources are used as radiation source (1).

11. Apparatus according to one of the preceding claims, **characterized in that** the detection of the intensity or absorption is carried out at the wavelengths $\lambda$=320 nm, $\lambda$=310, $\lambda$=305 nm, $\lambda$=300 nm, $\lambda$=290 nm, $\lambda$=280 nm, $\lambda$=270 nm, $\lambda$=266 nm, $\lambda$=260, $\lambda$=250 nm, $\lambda$=245 nm, $\lambda$=240, $\lambda$=235 nm, $\lambda$=230 nm, $\lambda$=220 nm and $\lambda$=205 nm.

**Revendications**

1. Un appareil pour le traitement extracorporel du sang ayant

- un dialyseur qui est séparé par une membrane semi-perméable dans une première et une deuxième chambre (29,30), où la première chambre (29) est disposée sur le trajet du dialysat et la deuxième chambre (30) est connectable à la circulation sanguine d'un patient au moyen d'une conduite d'arrivée du sang (32) et d'une conduite de sortie du sang (31),
- une entrée (20) du dialysat frais,
- une sortie (36) du dialysat utilisé,
- un dispositif de mesure (37) disposé dans la sortie (36), où le dispositif de mesure (37) a une source de rayonnement (1) émettant un rayonnement UV électromagnétique,
- où la source de rayonnement (1) est composée soit d'au moins deux sources de lumière monochromatique, soit d'au moins une source de lumière polychromatique avec des monochromateurs pour la génération d'un rayonnement UV monochromatique,
- une unité à microprocesseur (14), une unité de stockage, ainsi qu'une unité de sortie (15),

**caractérisé en ce que**
le dispositif de mesure (37) est conçu pour générer un rayonnement UV électromagnétique essentiellement monochromatique de longueurs d'onde différentes et le diriger à travers la sortie (36) du dialysat utilisé, où au moins un système de détection (5) est prévu pour la détection de l'intensité ou de l'absorption du rayonnement UV électromagnétique essentiellement monochromatique passant à travers la sortie (36) du dialysat utilisé et un système

d'équations $A_{\lambda i} = \sum_{j=1}^{n} A_j$ est déposé dans l'unité de stockage, dans lequel $A_{\lambda i}$ est l'absorption totale d'un mélange de composés à une longueur d'onde prédéterminée $\lambda_i$, $A_j$ est l'absorption d'un seul composé dans le mélange de composés et n et le nombre de composants intéressants dans le mélange de composés, qui contribuent à l'absorption.

2. L'appareil selon la revendication 1, **caractérisé en ce que** la source de rayonnement (1) composée de plusieurs sources de lumière est conçue pour l'émission d'un rayonnement électromagnétique dans la gamme allant de 180 nm à 380 nm.

3. L'appareil selon revendication 1 ou 2, **caractérisé en ce que** la source de rayonnement (1) a une pluralité de diodes électroluminescentes qui sont conçues de façon que chacune génère un rayonnement UV électromagnétique essentiellement monochromatique.

4. L'appareil selon revendication 1 ou 2, **caractérisé en ce que** la source de rayonnement (1) est conçue pour la génération d'un rayonnement électromagnétique polychromatique et des monochromateurs appropriés, en particulier des filtres optiques perméables seulement pour une spécifique longueur d'onde ou un filtre passe-bande avec plusieurs bandes passantes, sont prévus pour la génération d'un rayonnement électromagnétique essentiellement monochromatique.

5. L'appareil selon une des revendications précédentes, **caractérisé en ce que** l'unité à microprocesseur (14) est conçue pour résoudre le système d'équations $A_{\lambda i} = \sum_{j=1}^{n} A_j$ en utilisant les équations $A_{\lambda_i} = \varepsilon_{j,\lambda_i} \cdot l \cdot c_j$ (1) et les n points de mesure et l'absorption $A_1$ d'une substance de référence à une longueur d'onde de référence $\lambda_1$, où $A_j$ représente l'absorbance de la substance $j$ à une certaine longueur d'onde $\lambda_i$, $\varepsilon_{\lambda_i}$ représente le coefficient d'extinction dépendant de la longueur d'onde, l représente la longueur du trajet optique et $c_j$ représente la concentration de la substance respective.

6. L'appareil selon une des revendications 1 à 4, **caractérisé par le fait qu'**au moyen du microprocesseur (14) à l'aide d'une courbe d'absorption de référence de la substance de référence et du suivant système d'équations stocké dans l'unité de stockage

$$A_{\lambda i}(dialysat) \approx \frac{A_{\lambda 1}(\text{substance de référence})}{const_i} + \sum_{j=2}^{n} \varepsilon_{j,\lambda_i} \cdot l \cdot c_j$$
$$= \frac{A_{\lambda 1}(\text{substance de référence})}{const_i} + \sum_{j=2}^{n} A_j$$

où dans le dialysat utilisé, presque exclusivement la substance de référence absorbe à la longueur d'onde $\lambda_i$, où $const_i$ est le ratio de l'absorbance de la substance de référence pure à la longueur d'onde de référence $\lambda_1$ et l'absorbance de la substance de référence pure à une longueur d'onde $\lambda_j$, la détermination des absorptions et/ou des concentrations des toxines urémiques à la sortie (36) est simplifiée.

7. L'appareil selon la revendication 5 ou 6, **caractérisé en ce que** la substance de référence est choisie parmi le groupe comprenant les toxines urémiques UV actives : la créatinine, l'acide urique, l'acide hippurique, le sulfate d'indoxyle, le 4-hydroxynonenal, le malondialdéhyde, le p-crésol, le phénol, la protéine de liaison du rétinol et les fragments de ß2-microglobuline et/ou leurs combinaisons.

8. L'appareil selon la revendication 7, **caractérisé en ce que** l'acide urique est utilisé comme substance de référence.

9. L'appareil selon une des revendications 5 à 8, **caractérisé en ce que** la longueur d'onde de référence $\lambda_1$ est dans la gamme allant de 280 nm à 300 nm.

10. L'appareil selon une des revendications précédentes **caractérisé en ce que** 10 sources de lumière monochromatique sont utilisées comme source de rayonnement (1).

**11.** L'appareil selon une des revendications précédentes **caractérisé en ce que** la détection de l'intensité ou de l'absorption est effectuée aux longueurs: $\lambda$=320 nm, $\lambda$=310, $\lambda$=305 nm, $\lambda$=300 nm, $\lambda$=290 nm, $\lambda$=280 nm, $\lambda$=270 nm, $\lambda$=266 nm, $\lambda$=260, $\lambda$=250 nm, $\lambda$=245 nm, $\lambda$=240, $\lambda$=235 nm, $\lambda$=230 nm, $\lambda$=220 nm and $\lambda$=205 nm.

Figur 1

**Absorptionsspektrum von verbrauchtem Dialysat**

Figur 2

**Absorptionsspektrum von Harnsäure (c=1 mg/l)**

Figur 3

**Absorptionsspektrum von Malondialdehyd (c=1 mg/l)**

Figur 4

**Absorptionsspektrum von Creatinin   (c=1 mg/l)**

Figur 5

Original- und zusammengesetztes Spektrum des verbrauchten Dialysats

Figur 6

Figur 7

Figur 8

Figur 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1083948 B1
- DE 2934190 A1
- DE 69916053 T2
- US 5772606 A
- JP 02027264 A

**In der Beschreibung aufgeführte Nicht-Patentliteratur**

- **THEMA.** Haemodialysis Treatment monitored on-line by ultra violet absorbance. Linköping University Medical Dissertation No 962, 2006
- Lange's Nandbook of Chemistry. McGraw-Hill, Inc, 1992
- Second Handbook of Chemistry and Physics. CRC Press, 1975
- Third Practical Handbook of Biochemistry and Molecular Biology. CRC Press, 1992
- Peculiar character of dialyzate ultraviolet extinction spectra as an indicator of nucleic acid metabolism in humans. *Journal of Biomedical Optics,* Juli 2005, vol. 10 (4), 44026